# EUROPEAN PATENT SPECIFICATION

(11) **EP 4 267 238 B1**
(45) Date of publication and mention of the grant of the patent: **04.11.2026**
(21) Application number: 21844907.2
(22) Date of filing: 22.12.2021
(51) Int. Cl.: A61N 1/36, A61B 5/00, A61B 5/388

(54) **ELECTRICAL STIMULATION THERAPY**
ELEKTRISCHE STIMULATIONSTHERAPIE
THÉRAPIE PAR STIMULATION ÉLECTRIQUE

(30) Priority: 23.12.2020 US 202063130086 P; 13.01.2021 US 202163136948 P
(43) Date of publication of application: 01.11.2023
(73) Proprietor: Medtronic, Inc., Minneapolis, Minnesota 55432 (US)
(72) Inventor: DINSMOOR, David A., Minneapolis, Minnesota 55432 (US); VALLEJO, Ricardo, Minneapolis, Minnesota 55432 (US); SKERKER, Abigail Lauren, Minneapolis, Minnesota 55432 (US); LITVAK, Leonid M., Minneapolis, Minnesota 55432 (US); HAGEMAN, Kristin N., Minneapolis, Minnesota 55432 (US); BINK, Hank, Minneapolis, Minnesota 55432 (US)
(74) Representative: Zimmermann & Partner Patentanwälte mbB
(86) International application number: PCT/US2021/064833
(87) International publication number: WO 2022/140529

(56) References cited:
- WO-A1-2007/117232
- US-A1- 2019 388 692
- US-A1- 2019 388 695
- US-A1- 2020 101 294

## Description

This application claims the benefit of U.S. Provisional Patent Application No. 63/130,086, filed December 23, 2020 and U.S. Provisional Patent Application No. 63/136,948, filed January 13, 2021.

### TECHNICAL FIELD

This disclosure generally relates to electrical stimulation therapy, and more specifically, control of electrical stimulation therapy.

### BACKGROUND

Medical devices may be external or implanted and may be used to deliver electrical stimulation therapy to patients via various tissue sites to treat a variety of symptoms or conditions such as chronic pain, tremor, Parkinson's disease, epilepsy, urinary or fecal incontinence, sexual dysfunction, obesity, or gastroparesis. A medical device may deliver electrical stimulation therapy via one or more leads that include electrodes located proximate to target locations associated with the brain, the spinal cord, pelvic nerves, peripheral nerves, or the gastrointestinal tract of a patient. Stimulation proximate the spinal cord, proximate the sacral nerve, within the brain, and proximate peripheral nerves are often referred to as spinal cord stimulation (SCS), sacral neuromodulation (SNM), deep brain stimulation (DBS), and peripheral nerve stimulation (PNS), respectively.

Electrical stimulation therapy may be delivered by the medical device in a train of electrical stimulation pulses, and parameters that define the electrical stimulation pulses may include a frequency, an amplitude, a pulse width, and a pulse shape. Documents US 2019/388692 A1 and US 2019/388695 A1 relate to devices for electrical stimulation therapy.

### SUMMARY

The invention is defined by the appended claims. Systems, devices, and techniques are described for controlling the delivery of electrical stimulation therapy delivered to a patient, e.g., to treat a patient condition.

Methods disclosed hereinafter are not claimed and only of exemplary nature to show how the system of the present invention can be applied.

In some examples, the electrical stimulation therapy may include a plurality of electrical stimulation pulses delivered to the patient as a pulse train. For example, the pulse train may include a plurality of first pulses interleaved with a plurality of second pulses, e.g., delivered with one or more first pulses alternating with one or more a second pulses. Each of the first pulses and second pulses may include a first phase having a first polarity and a second phase having the opposite polarity from the first phase, and one or more parameters defining the first and second phases may be selected such that each pulse is substantially charged balanced (e.g., with the charge of the first phase being equal and opposite of the second phase). In some examples, an interphase interval of some amount of time may be present between the respective phases for a pulse, e.g., rather than delivering the second phase directly after the first phase without any delay.

In some examples, the first pulses of the pulse train may function as control pulses, which are delivered in conjunction with a plurality of informed pulses that are at least partially interleaved with the control pulses. The control pulses may be stimulation pulses that are configured to elicit an electrical signal, e.g., as a detectable evoked compound action potential (ECAP) signal. In some examples, control pulses may contribute to the therapy for a patient. In other examples, control pulses do not contribute to the therapy for the patient, e.g., non-therapeutic pulses. In this manner, control pulses may or may not be configured to elicit a therapeutic effect for the patient. Informed pulses may be stimulation pulses that are at least partially defined by one or more parameters based on the detectable ECAP signal elicited from one or more control pulses. In this manner, the informed pulses are "informed" by the ECAP signal detected from a control pulse. Informed pulses are also configured to provide a therapy to a patient, such as paresthesia that relieves pain symptoms. A medical device (e.g., an implantable medical device) may deliver one or more control pulses to the patient via one or more leads, and the system may sense the resulting ECAP signal elicited by the control pulse(s) - all between consecutive informed pulses. For example, in response to determining that a characteristic of the ECAP signal (e.g., a voltage amplitude) elicited by a control pulse has deviated from a target ECAP characteristic, the system may change one or more stimulation parameters of the next one or more informed pulses to be delivered to the patient.

The pulse train may include a second pulses that are interleaved with the first pulses. In some examples, the second pulses of the pulse train that are interleaved with the first pulses may elicit an electrical signal. However, unlike that of the first pulses, a medical device may not deliver one or more informed pulses based on a signal elicited by the second pulses. Rather, informed pulses that are delivered directly following a second pulse of the pulse train may be "informed" based on the first pulse directly preceding the second pulse. The first and second pulses of the pulse train delivered by a medical device may not be identical to each other. For example, the first pulses of the pulse train may have both an active first phase and an active second phase (referred to in some cases as an active recharge) while the second pulses of the pulse train may have an active first phase and a passive second phase (referred to in some cases as a passive recharge). Put another way, in some examples, the first and second pulses are part of the same pulse train delivered at a substantially continuous/constant frequency over a period of time but the second pulses may not be the same as the first pulses because a medical device does not sense the response elicited by the second pulses. Additionally, or alternatively, one or more of the plurality of second pulses may have an interphase interval that is longer than an interphase interval of the first pulses. Additionally, or alternatively, the second pulses may alternate between pulses having a cathode active phase and an anodic active phase according to a ratio (e.g., with the first pulses may all having a first active phase of the same polarity).

Additionally, or alternatively, in some examples, the electrical stimulation therapy may include a pulse train delivered before and/or after sensing of an electrical signal elicited by the delivery of electrical stimulation to a patient. For example, an IMD may deliver a pulse train of informed pulses and sense an electrical signal (e.g., an ECAP) elicited by the delivery of one or more control pulses. The pulse train may include one or more first pulses at least partially interleaved with one or more second pulses. The first pulse(s) may include a cathodic active first phase and a passive second phase, and the second pulse(s) include an anodic first phase and passive second phase (e.g., as compared to a pulse train in which all the pulse have an active first phase of the same polarity and a passive second phase of the opposite polarity and/or all the pulses have an active first phase and an active second phase). The pulse train may be followed by the delivery of one or more additional control pulses that again elicit an electrical signal in response to the delivered stimulation after which another train of informed pulses is delivered. In some examples, the alternating polarity of the pulse train may be employed to limit stimulation artifacts and maintain electrochemical balance (e.g., at the tissue site at which the stimulation is delivered to the patient), without the battery capacity draw associated with a train of pulses having an active second (recharge) phase. In some examples, the reduced charge build up associated with the train of informed pulses may improve the sensing of electrical signals elicited after delivery of the train of informed pulses.

In some examples, the IMD may be configured to deliver the train of informed pulses with alternating polarity for the active first phase and passive second phase (e.g., on a 1:1 or 2:2 pulse basis) after each control pulse (or after each informing control pulse). In other examples, the IMD may be configured to adjust the train of control pulse from a train of pulses all of the same polarity first phase to a train of pulses with alternating polarity first phase and passive second phase based on one or more parameters of the sensed electrical signal(s) elicited by the control pulse(s). For example, the IMD may make such an adjustment when the slope of a linear trend for a plurality of sensed ECAPs elicited by a control pulse is determined to be above a predetermined threshold. This may allow for the IMD to maintain desirable sensing of ECAPs or other electrical signals elicited by control pulses over time.

In one example, the disclosure relates to a method comprising delivering, via a medical device, a pulse train at a frequency to a patient, the pulse train comprising a plurality of first pulses at least partially interleaved with a plurality of second pulses, wherein the plurality of first pulses are configured to facilitate sensing elicited electrical signals, each pulse of the plurality of first pulses having an active first phase and active second phase, and wherein each pulse of the plurality of second pulses comprises an active first phase and a passive second phase.

In another example, the disclosure relates to a system comprising stimulation generation circuitry configured to deliver a pulse train at a frequency to a patient, the pulse train comprising a plurality of first pulses at least partially interleaved with a plurality of second pulses, wherein the plurality of first pulses are configured to facilitate sensing elicited electrical signals, each pulse of the plurality of first pulses having an active first phase and active second phase, and wherein each pulse of the plurality of second pulses comprises an active first phase and a passive second phase.

In another example, the disclosure relates to a computer-readable storage medium comprising instructions that, when executed, cause processing circuitry to: control delivery of a pulse train at a frequency to a patient, the pulse train comprising a plurality of first pulses interleaved with a plurality of second pulses, wherein the plurality of first pulses are configured to facilitate sensing elicited electrical signals, each pulse of the plurality of first pulses having an active first phase and active second phase, and wherein each pulse of the plurality of second pulses comprises an active first phase and a passive second phase.

In another example, the disclosure relates to a method comprising delivering, via a medical device, a pulse train at a frequency to a patient, the pulse train comprising a plurality of first pulses at least partially interleaved with a plurality of second pulses, wherein the plurality of first pulses are configured to facilitate sensing elicited electrical signals, each pulse of the plurality of first pulses having an active first phase and active second phase, and wherein the plurality of second pulses alternate between a cathodic active first phase and an anodic active first phase according to a ratio.

In another example, the disclosure relates to a method comprising delivering, via a medical device, a pulse train at a frequency to a patient, the pulse train comprising a plurality of first pulses at least partially interleaved with a plurality of second pulses, wherein the plurality of first pulses are configured to facilitate sensing elicited electrical signals, each pulse of the plurality of first pulses having an active first phase and active second phase, and wherein at least one pulse of the plurality of second pulses has an interphase interval that is longer than an interphase interval of at least one pulse of the plurality of first pulses.

In another example, the disclosure relates to a method comprising delivering, via a medical device, a pulse train to a patient, the pulse train comprising at least one first pulse at least partially interleaved with at least one second pulse, wherein the at least one first pulse includes a cathodic active first phase and a passive second phase, and wherein the at least one second pulse includes an anodic active first phase and passive second phase; and subsequently sensing, via sensing circuitry, an electrical signal elicited by delivery of electrical stimulation to a patient.

In another example, the disclosure relates to a system comprising sensing circuitry; a stimulation generator; and processing circuitry, the processing circuitry configured to: control the stimulation generator to deliver, a pulse train to a patient, the pulse train comprising at least one first pulse at least partially interleaved with at least one second pulse, wherein the at least one first pulse includes a cathodic active first phase and a passive second phase, and wherein the at least one second pulse includes an anodic first phase and passive second phase, and sense, via the sensing circuitry, an electrical signal elicited by electrical stimulation delivered to the patient from the stimulation generator.

In another example, the disclosure relates to a computer-readable storage medium comprising instructions that, when executed, cause processing circuitry to: control delivery, via a medical device, a pulse train to a patient, the pulse train comprising at least one first pulse at least partially interleaved with at least one second pulse, wherein the at least one first pulse includes a cathodic active first phase and a passive second phase, and wherein the at least one second pulse includes an anodic active first phase and passive second phase; and subsequently sense, via sensing circuitry, an electrical signal elicited by delivery of electrical stimulation to a patient.

The details of one or more examples of the techniques of this disclosure are set forth in the accompanying drawings and the description below. Other features, objects, and advantages of the techniques will be apparent from the description and drawings, and from the claims.

### BRIEF DESCRIPTION OF DRAWINGS

FIG. 1 is a conceptual diagram illustrating an example system that includes a medical device programmer and an implantable medical device (IMD) configured to deliver spinal cord stimulation (SCS) therapy according to the techniques of the disclosure.
FIG. 2A is a block diagram of the example IMD of FIG. 1.
FIG. 2B is a block diagram of the example external programmer of FIG. 1.
FIG. 3 is a timing diagram illustrating an example of electrical stimulation pulses and respective sensed ECAPs, in accordance with one or more techniques of this disclosure.
FIG. 4 is an example timing diagram illustrating an example informing control pulse and non-informing control pulse, in accordance with some examples of the disclosure.
FIG. 5 is a timing diagram illustrating an example train of control pulses, in accordance with one or more techniques of this disclosure.
FIGS. 6A-6C are example circuit diagram illustrating anodic active stimulation and passive anodic stimulation, in accordance with some examples of the disclosure.
FIG. 7 is a flowchart illustrating an example technique for therapy delivery according to the techniques of this disclosure.
FIG. 8 is a flowchart illustrating another example technique for therapy delivery according to the techniques of this disclosure.
FIG. 9 is a flowchart illustrating another example technique for therapy delivery according to the techniques of this disclosure.
FIGS. 10-15 are timing diagrams illustrating example electrical stimulation patterns in accordance with some examples of this disclosure.
FIGS. 16 and 17 are flow diagrams illustrating example techniques for delivering therapy according to techniques of this disclosure.
FIGS. 18 and 19 are diagrams comparing the slope of linear trend for ECAPs following two different examples trains of pulse.
FIGS. 20-22 are timing diagrams illustrating example electrical stimulation patterns in accordance with some examples of this disclosure.
FIGS. 23A and 23B are timing diagrams illustrating phases of example electrical stimulation pulses accordance with some examples of this disclosure.

### DETAILED DESCRIPTION

The disclosure is directed to devices, systems, and techniques for delivering electrical stimulation to a patient. In some examples, the disclosure is directed to devices, systems, and techniques for delivering electrical stimulation to a patient where the electrical stimulation includes a pulse train with first pulses configured to elicited sensed electrical signals as well as second pulses. As described above, one type of electrical signal elicited by the first pulses may be ECAPs. For ease of description, examples of the disclosure are primarily described with regard to the elicited signals being ECAPs although other types of sensed elicited electrical signals such as evoked compound muscle action potentials (eCMAPs) or local field potentials (LFPs) are contemplated. As will be described below, in some examples, the second pulses of the pulse train at least partially interleaved with the first pulses may elicit an ECAP. However, the IMD may not sense the ECAPs elicited by the second pulses like the IMD senses the ECAPs of the first pulses. Accordingly, the second pulses may differ in one or more ways compared to that of the first pulses, e.g., in order to reduce power consumption resulting from the delivery of the second pulses compared to the first pulses. For example, the second pulses may have a passive second phase, the second pulses may have a longer interphase interval between first and second phases, the second pulses may have lower amplitude, and/or the first phases of the second pulses may alternate polarity).

In some examples, an IMD may be configured to automatically adjust electrical stimulation therapy delivered to a patient based on one or more characteristics of evoked compound action potentials (ECAPs) received by a medical device in response to control stimulation pulses delivered by the medical device. Electrical stimulation therapy is typically delivered to a target tissue (e.g., one or more nerves or muscle) of a patient via two or more electrodes. Parameters of the electrical stimulation therapy (e.g., electrode combination, voltage or current amplitude, pulse width, pulse frequency, etc.) are selected by a clinician and/or the patient to provide relief from various symptoms, such as pain, muscle disorders, etc. However, as the patient moves, the distance between the electrodes and the target tissues changes. Since neural recruitment is a function of stimulation intensity and distance between the target tissue and the electrodes, movement of the electrode closer to the target tissue may result in increased perception by the patient (e.g., possible painful sensations), and movement of the electrode further from the target tissue may result in decreased efficacy of the therapy for the patient.

ECAPs are a measure of neural recruitment because each ECAP signal represents the superposition of electrical potentials generated from axons firing in response to an electrical stimulus (e.g., a stimulation pulse). Changes in a characteristic (e.g., an amplitude of a portion of the signal, an area under one or more peaks, frequency content, and/or maximum and/or minimum peak timing) of an ECAP signals occur as a function of how many axons have been activated by the delivered stimulation pulse. A system can monitor changes in the characteristic of the ECAP signal and use that change in the characteristic to adjust one or more stimulation parameter of the informed pulses delivered to the patient. For example, the system can reduce the intensity of informed pulses (e.g., reduce a current amplitude and/or pulse width) in response to detecting an increase in an amplitude of an ECAP signal elicited by a control pulse.

Using such an approach, a medical device may be configured to deliver a plurality of informed pulses configured to provide a therapy to the patient which may be adjusted or otherwise controlled based on one or more parameters of ECAP signals elicited by previously delivered control pulses. It may be desirable for the individual control pulses that are sensed by an IMD to "inform" the informed pulses to have an active first phase followed by an active second phase (active recharge) with substantially the same amplitude and with a relatively small interphase interval between the two phases of the pulse. In some examples, the control pulses may be delivered at a frequency that is substantially the same as the control pulses such that each informed pulse is "informed" by the ECAP elicited by a directly preceding control pulse. For example, a train of control pulses delivered at a rate of 50 Hz may be interleaved with a train of informed pulses also delivered at 50 Hz. Examples of medical devices configured to deliver control pulses and informed pulses in such a manner may include one or more of the examples described in U.S. Patent Application No. 16/449,152, filed June 21, 2019, the entire content of which is incorporated herein by reference.

However, while an IMD may deliver a train of control pulses to a patient over a period of time at the same frequency as informed pulses so that each informed pulse is "informed" by an ECAP elicited by a respective control pulse, during other periods of time, an IMD may not sense and adjust, if warranted, the informed pulses on a pulse-by-pulse basis. For example, the rate at which ECAPs are sensed for purposes of "informing" informed pulses may be reduced when a patient is relatively immobile (e.g., sleeping) even though the rate at which the informed pulses are delivered stays constant. As one example, an IMD may be configured to only sense an ECAP elicited by a control pulse and adjust, if warranted, subsequently delivered informed pulses on an every other pulse basis over a given period of time. In such as example, during that period of time, an IMD may be configured to sense an ECAP elicited by a control pulse and adjust, if warranted, a subsequently delivered informed pulse and then deliver the next "informed" pulse so that it is the same as the first informed pulse rather than sensing the ECAP of the next control pulse in the train of control pulses and adjusting that second informed pulse if necessary. In other examples, an IMD may only sense and adjust, if warranted, every third "informed" pulse (or even greater than every third "informed" pulse) in a train of informed pulses based on the ECAP elicited by a control pulse.

In some examples, when the rate at which the informed pulses are adjusted based on a control pulse, that rate at which the control pulses are delivered may be reduced to match the rate at which the ECAPs are sensed by the IMD. For example, during a period when the informed pulses are only adjusted based on sensed ECAPs elicited by a control pulse on an every other pulse basis, the rate at which the control pulses are delivered may be reduced to half that of the rate of the informed pulse delivery. As one specific example, over a first period of time, an IMD may deliver a series of control pulses delivered at a frequency of 50 Hz and also deliver informed pulses at a frequency of 50 Hz, where the informed pulses are interleaved with the control pulses. During a second period of time (e.g., when a patient is determined to be sleeping or otherwise sedentary), the IMD may only sense an ECAP signal elicited by a control pulse and adjust, if warranted, every other informed pulse so that the control pulses may only need to be delivered at 25 Hz.

However, that nature of the control pulse may be such that a patient may perceive each control pulse, or a sensation caused by control pulses at a certain frequency, and thus perceive any changes to the rate the control pulses are delivered by an IMD. Thus, to maintain a substantially constant perception of the control pulses by the patient, it may be desirable to maintain the delivery of the train of control pulses at substantially constant rate even though the system may not use every ECAP elicited by each control pulse to inform an informed pulse or even enable sensing circuitry after control pulses from which no ECAP will attempt to be sensed, e.g., for those reasons described above.

In accordance with examples of the disclosure, an IMD (or other medical device) may be configured to deliver a train of control pulses at a given rate, where all the respective pulses in the train of control pulses are not exactly the same. For example, such a pulse train may include first pulses that are configured to facilitate sensing of elicited signals (e.g., by eliciting an ECAP) interleaved with second pulses that are different from the first pulses in one or more respects. The second pulses may be those pulses which are not used by the IMD or other medical device to "inform" an informed pulse, e.g., for those reasons described above. While the second pulses in the train of control pulses may still elicit a response such as an ECAP, the IMD may not actively sense electrical signals after delivering the second pulses and/or control subsequently delivered informed pulses based on any sensed signals. Rather, the informed pulses delivered after the second pulses may be "informed" based on a previously delivered first pulse. For ease of description, such second pulses in the pulse train may also be referred to as "non-informing control pulses" or "non-informing pulses" while the first pulses in the pulse train may also be referred to as "informing control pulses" or "informing pulses." Since the IMD may not use any sensed signals evoked from the second pulses, one or more aspects of the second pulses may be modified to achieve a different purpose, such as reduce power consumption.

As will be described below, an IMD may be configured to deliver the pulse train such that the first pulses are interleaved with second pulses (e.g., using the same stimulation vector). For example, the IMD may deliver the pulse train such that a single first pulse is followed by a single second pulse, followed by a single first pulse, followed by a single second pulse, and so forth. Such a pulse train may have a 1:1 ratio of first pulses to second pulses. In another example, the IMD may deliver the pulse train such that there is a 1:2 ratio of first pulses to second pulses (e.g., by repeatedly delivering a single first pulse followed by two second pulses). Any suitable ratio of first pulses to second pulse is contemplated. Additionally, the ratio of first pulses to second pulses in the pulse train may vary over time, e.g., so that the rate at which the informing control pulses (first pulses) matches the rate at which the IMD is delivering informed pulses based on the informing control pulses.

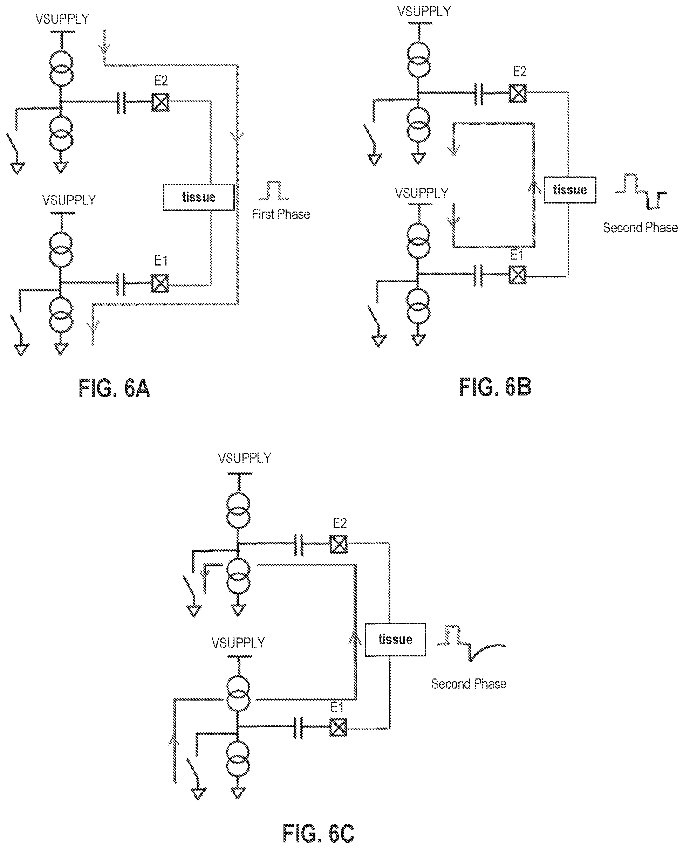

In some examples, the first pulses of the train of control pulses may each have an active first phase and an active second phase (e.g., for an active recharge). Using an active second phase for informing control pulses may be preferred because the active recharge clear the charge from the first phase relatively quickly, e.g., to minimize the amount of the control pulse that overlaps in time with the ECAP. Conversely, since the second pulses as non-informing pulses, each second pulse may have an active first phase and a passive second phase (e.g., for a passive recharge). Put another way, interference of a longer passive second phase with an evoked ECAP has no consequence because the ECAP is not used for any purpose by the IMD. Using such a pulse train, a patient may experience a substantially consistent perception of the pulse train (e.g., with the perception of the first and second pulses being the same to the patient) but with power savings associated with passive second phase of the second pulses in the pulse train over using an active second phase. For example, for two pulses having the same total charge in each phase, a pulse having an active first phase and passive second phase may require less power than a pulse having an active first phase and an active second phase. This is due at least in part to the passive second phase merely requiring grounding of the electrodes to passively reduce excess charge or "recharge" the tissue and associated electrode blocking capacitors (like those shown in FIG. 6, e.g.) to approximately neutral charge. Conversely, an active phase requires the IMD to pull charge from the battery in order to deliver the active phase of the pulse. Thus, by delivering a pulse train that has at least some second pulses with a passive recharge in addition to first pulses having an active second phase, the IMD may conserve power relative to substantially the same pulse train but with all the control pulses having an active second phase.

Additionally, or alternatively, an IMD or other medical device may deliver a train of control pulses including both first pulses and second pulses, where the interphase interval for the respective second pulses is longer than the interphase interval for the first pulses. For example, the interphase of the respective second pulses may be increased compared to that of the first pulses in order to limit blocking of the action potential initiated by the first phase, and subsequently lower the stimulation amplitude needed for neural activation. In some examples, the longer interphase interval enables the first phase to have a lower amplitude than with shorter interphase intervals. With shorter intervals, the second phase may end up removing some of the charge from the first phase. Therefore, the amplitude may need to be higher with shorter interphase intervals in order to get the appropriate charge to the tissue.

In some examples, the interphase interval of the second pulses may be longer than the interphase interval of the first pulses when the second pulses also include a passive second phase (passive recharge). Similarly, the interphase interval of the second pulses may be longer than the interphase interval of the first pulses when the second pulses have an active second phase. Lengthening the interphase interval for the second pulses may effectively allow the amplitude first phase of the second pulses to be reduced, thus reducing the power consumption from the second pulses, e.g., as compared to higher amplitude first pulses. For example, the use of longer interphase interval may enable the system to reduce the amplitude so the IMD does not have to overcompensate for the second phase removing charge too early (e.g., since the ECAP elicited by the second pulse is not being sensed by IMD). Conversely, the activation and/or perception threshold for the first pulses having active first and second phases may be higher because a short interphase interval requires a higher first phase amplitude to achieve the threshold because the second phase delivered after a relatively short interphase interval may "strip away" some depolarization away from the first phase. In some examples, the second phase is delivered to ensure charge balance to prevent build-up of charge in a tissue. If the second phase is delivered too early, it may act to inhibit the action potential which was kicked off by the first phase. This may result in higher stimulation amplitudes needed to get the desired result.

Additionally, or alternatively, an IMD or other medical device may deliver a train of control pulses including both first pulses and second pulses, where the second pulse alternate between a cathodic first phase and an anodic first phase according to a ratio. For example, the ratio of cathodic first phase to anodic first phase may be 1:1 such that the first phase of respective second pulses alternates between anodic phase and cathodic phase every single second pulse. Alternating between a cathodic first phase and anodic first phase may reduce physiological effects of the pulse train on the patient (e.g., by reducing the level of glutamate and/or other substances or neurotransmitters) that may otherwise occur when the second pulses all have the same polarity first phase (e.g., all having a cathodic active first phase with anodic passive second phase). In some examples, the ratio of cathodic first phase and an anodic first phase may be varied over a period of time. For example, an IMD may be configured to adjust such a ratio based on a level of glutamate and/or other substance sensed by the IMD during the delivery of the pulse train, e.g., on a closed loop basis. As another example, the IMD may be configured to adjust such a ratio based on one or more components of the sensed ECAP elicited by the first pulses, where the one or more components are indicative of the physiological state of the patient. As another example, the IMD may be configured to adjust such a ratio based on the level of charge of a power source of the IMD (e.g., to reduce the power consumption when the power source level of charge is below a threshold amount), an accelerometer signal (e.g., to indicate a posture and/or activity of the patient), gyroscope signal (e.g., to indicate a posture and/or activity of the patient), or a time of day.

In addition to, or as an alternative to, the examples of the disclosure described herein with regard to the delivery of control pulse trains, examples of the disclosure also relate to the delivery of pulse trains, such as a train of control pulses, before the sensing of electrical signals elicited by electrical stimulation. In the case of a train of informed pulses delivered following a control pulse, the informed pulse train may contribute to the therapy for a patient, e.g., by treating a patient condition, and may be adjusted based on the sensed electrical signal as described herein. As will be described below, the informed pulse train may include at least one first pulse at least partially interleaved with at least one second pulse, where the at least one first pulse includes a cathodic active first phase and a passive second phase, and where the at least one second pulse includes an anodic first phase and passive second phase.

In some instances, a train of informed pulses may be delivered to chronically activate neural tissue in which the pulses in the pulse train are all generally biphasic with active first and second phases, e.g., as compared to monophasic stimulation with an active first phase and passive second phase, with the first phase of each pulse being the same polarity. The first phase of stimulation of the biphasic stimulation pair may serve to activate the tissue at the working electrode, while the second phase serves to reverse electrochemistry or reaction products which formed during the first phase. However, such a train of biphasic pulses may require a relatively large draw on the battery of an IMD, e.g., on account of the active nature of the second phase. The modification to deliver such a train of pulses with a passive second phase but delivering the pulses so that each pulse has an active first phase with the same polarity may result in charge buildup and an undesirable effect on biopotential sensing, such as the sensing of electrical signals elicited by one or more control pulses delivered following the delivery of the informed pulse train, e.g., due to charge build up near one or more of the stimulation electrodes.

In accordance with some examples the disclosure, an IMD may be configured to deliver a train of pulses, such as a train of informed pulses, to a patient prior to the sensing of electrical signals elicited by electrical stimulation delivered to the patient. The pulse train may include at least one first pulse at least partially interleaved with at least one second pulse, where the at least one first pulse includes a cathodic active first phase and a passive second phase, and where the at least one second pulse includes an anodic first phase and passive second phase. In some examples, the pulses alternative polarity on a one pulse to one pulse basis while in other examples other alternating patterns are contemplated (e.g., alternating between two pulses of the same polarity of the first active phase and two pulses of the opposite polarity for the first active phase). The described stimulation patterns for the pulse trains may allow for individual pulses having passive recharge phases (e.g., passive anodic stimulation) while reducing the negative effects on biopotential sensing. In some examples, the described patterns allow for less current draw (e.g., power savings) than a pulse train employing active second phase (active recharge) and/or more capability for biopotential sensing due to reduced charge build-up on electrodes.

Although electrical stimulation is generally described herein in the form of electrical stimulation pulses, electrical stimulation may be delivered in non-pulse form in other examples. For example, electrical stimulation may be delivered as a signal having various waveform shapes, frequencies, and amplitudes. Therefore, electrical stimulation in the form of a non-pulse signal may be a continuous signal than may have a sinusoidal waveform or other continuous waveform.

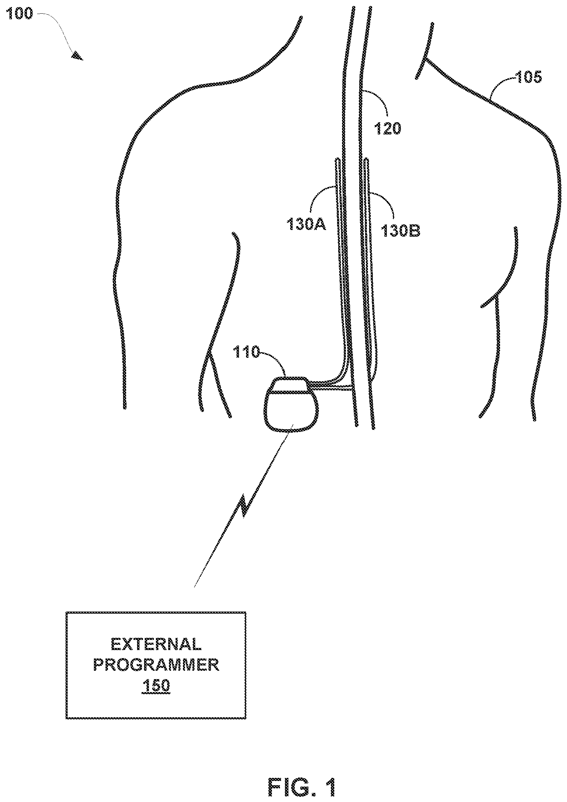

FIG. 1 is a conceptual diagram illustrating example system 100 that includes implantable medical device (IMD) 110 configured to deliver electrical stimulation therapy to patient 105 in accordance with examples of the disclosure. In the example shown in FIG. 1, IMD 110 is configured to deliver spinal cord stimulation (SCS) therapy according to the techniques of the disclosure. Although the techniques described in this disclosure are generally applicable to a variety of medical devices including external and implantable medical devices (IMDs), application of such techniques to IMDs and, more particularly, implantable electrical stimulators (e.g., neurostimulators) will be described for purposes of illustration. More particularly, the disclosure will refer to an implantable spinal cord stimulation (SCS) system for purposes of illustration, but without limitation as to other types of medical devices or other therapeutic applications of medical devices.

As shown in FIG. 1, system 100 includes an IMD 110, leads 130A and 130B, and external programmer 150 shown in conjunction with a patient 105, who is ordinarily a human patient. In the example of FIG. 1, IMD 110 is an implantable electrical stimulator that is configured to generate and deliver electrical stimulation therapy to patient 105 via one or more electrodes of electrodes of leads 130A and/or 130B (collectively, "leads 130"), e.g., for relief of chronic pain or other symptoms. In other examples, IMD 110 may be coupled to a single lead carrying multiple electrodes or more than two leads each carrying multiple electrodes. IMD 110 may be configured to generate and deliver control pulses configured to elicit ECAP signals that may or may not contribute to the therapy of informed pulses. The control pulses may be non-therapeutic in some examples. As described herein, the train of control pulses may include both informing control pulses (first pulses) and non-informing control pulses (second pulses). The informing control pulses may be different from the non-informing control pulses, e.g., with the informing pulses having an active second phase and the non-informing control pulses having a passive second phase, the non-informing pulses having a longer interphase interval than the informing pulses, and/or the non-informing pulses alternating between an anodic first active phase and a cathodic first active phase. Each of the techniques for changing the non-informing control pulses compared to the informing control pulses described herein (e.g., a passive second phase for the non-informing pulses, alternating the polarity of the active first phase of the non-informing pulses, a longer interphase interval for non-informing pulses, and a lower amplitude for non-informing pulses) may be employed individually or in combination with each other.

IMD 110 may be configured to sense electrical signals elicited by the delivered \ control pulses via one or more electrodes on leads 130 and deliver a train of informed pulses including at least one first pulse at least partially interleaved with at least one second pulse, where the at least one first pulse includes a cathodic active first phase and a passive second phase, and where the at least one second pulse includes an anodic first phase and passive second phase.

IMD 110 may be a chronic electrical stimulator that remains implanted within patient 105 for weeks, months, or even years. In other examples, IMD 110 may be a temporary, or trial, stimulator used to screen or evaluate the efficacy of electrical stimulation for chronic therapy. In one example, IMD 110 is implanted within patient 105, while in another example, IMD 110 is an external device coupled to percutaneously implanted leads. In some examples, IMD 110 uses one or more leads, while in other examples, IMD 110 is leadless.

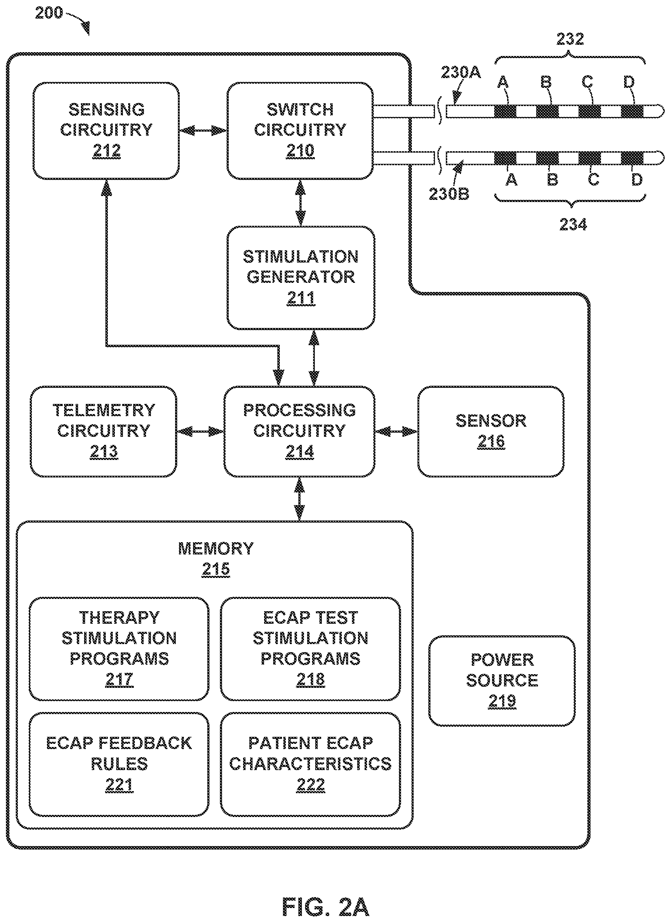

IMD 110 may be constructed of any polymer, metal, or composite material sufficient to house the components of IMD 110 (e.g., components illustrated in FIG. 2A) within patient 105. In this example, IMD 110 may be constructed with a biocompatible housing, such as titanium or stainless steel, or a polymeric material such as silicone, polyurethane, or a liquid crystal polymer, and surgically implanted at a site in patient 105 near the pelvis, abdomen, or buttocks. In other examples, IMD 110 may be implanted within other suitable sites within patient 105, which may depend, for example, on the target site within patient 105 for the delivery of electrical stimulation therapy. The outer housing of IMD 110 may be configured to provide a hermetic seal for components, such as a rechargeable or non-rechargeable power source. In addition, in some examples, the outer housing of IMD 110 may be selected from a material that facilitates receiving energy to charge the rechargeable power source.

Electrical stimulation energy, which may be constant current or constant voltage-based pulses, for example, is delivered from IMD 110 to one or more target tissue sites of patient 105 via one or more electrodes (not shown) of implantable leads 130. In the example of FIG. 1, leads 130 carry electrodes that are placed adjacent to the target tissue of spinal cord 120. One or more of the electrodes may be disposed at a distal tip of a lead 130 and/or at other positions at intermediate points along the lead. Leads 130 may be implanted and coupled to IMD 110. The electrodes may transfer electrical stimulation generated by an electrical stimulation generator in IMD 110 to tissue of patient 105. Although leads 130 may each be a single lead, lead 130 may include a lead extension or other segments that may aid in implantation or positioning of lead 130. In some other examples, IMD 110 may be a leadless stimulator with one or more arrays of electrodes arranged on a housing of the stimulator rather than leads that extend from the housing. In addition, in some other examples, system 100 may include one lead or more than two leads, each coupled to IMD 110 and directed to similar or different target tissue sites.

The electrodes of leads 130 may be electrode pads on a paddle lead, circular (e.g., ring) electrodes surrounding the body of the lead, conformable electrodes, cuff electrodes, segmented electrodes (e.g., electrodes disposed at different circumferential positions around the lead instead of a continuous ring electrode), any combination thereof (e.g., ring electrodes and segmented electrodes) or any other type of electrodes capable of forming unipolar, bipolar or multipolar electrode combinations for therapy. Ring electrodes arranged at different axial positions at the distal ends of lead 130 will be described for purposes of illustration.

The deployment of electrodes via leads 130 is described for purposes of illustration, but arrays of electrodes may be deployed in different ways. For example, a housing associated with a leadless stimulator may carry arrays of electrodes, e.g., rows and/or columns (or other patterns), to which shifting operations may be applied. Such electrodes may be arranged as surface electrodes, ring electrodes, or protrusions. As a further alternative, electrode arrays may be formed by rows and/or columns of electrodes on one or more paddle leads. In some examples, electrode arrays may include electrode segments, which may be arranged at respective positions around a periphery of a lead, e.g., arranged in the form of one or more segmented rings around a circumference of a cylindrical lead. In other examples, one or more of leads 130 are linear leads having 8 ring electrodes along the axial length of the lead. In another example, the electrodes are segmented rings arranged in a linear fashion along the axial length of the lead and at the periphery of the lead.

The stimulation parameter of a therapy stimulation program that defines the stimulation pulses of electrical stimulation therapy by IMD 110 through the electrodes of leads 130 may include information identifying which electrodes have been selected for delivery of stimulation according to a stimulation program, the polarities of the selected electrodes, i.e., the electrode combination for the program, and voltage or current amplitude, pulse frequency, pulse width, pulse shape of stimulation delivered by the electrodes. These stimulation parameters of informed pulses are typically predetermined parameter values determined prior to delivery of the informed pulses. However, in some examples, system 100 may change one or more parameter values automatically based on one or more factors or based on user input. In some examples, a therapy stimulation program may define the delivery of a train of informed pulses so that the train includes at least one first pulse at least partially interleaved with at least one second pulse, where the at least one first pulse includes a cathodic active first phase and a passive second phase, and where the at least one second pulse includes an anodic first phase and passive second phase.

In addition to informed stimulation pulses, an ECAP test stimulation program may define stimulation parameter values that define both non-informing and informing control pulses delivered by IMD 110 through at least some of the electrodes of leads 130. These stimulation parameter values may include information identifying which electrodes have been selected for delivery of the non-informing and informing control pulses, the polarities of the selected electrodes, i.e., the electrode combination for the program, and voltage or current amplitude, pulse frequency, pulse width, and pulse shape of stimulation delivered by the electrodes. As described herein, one or more stimulation parameter values for non-informing control pulses may be different than one or more stimulation parameter values of informing control pulses.

The stimulation signals (e.g., one or more stimulation pulses or a continuous stimulation waveform) defined by the parameters of each ECAP test stimulation program are configured to evoke a compound action potential from nerves. In some examples, the ECAP test stimulation program may define when the non-informing and informing control pulses are to be delivered to the patient based on the frequency and/or pulse width of the informed pulses. However, the stimulation defined by each ECAP test stimulation program may or may not be intended to provide or contribute to therapy for the patient. In an example where the non-informing and/or informing control pulses contribute to or provide therapy for the patient, the ECAP test stimulation program may also be used in place of, or be the same as, a therapy stimulation program.

Although FIG. 1 is directed to SCS therapy, e.g., used to treat pain, in other examples system 100 may be configured to treat any other condition that may benefit from electrical stimulation therapy. For example, system 100 may be used to treat tremor, Parkinson's disease, epilepsy, a pelvic floor disorder (e.g., urinary incontinence or other bladder dysfunction, fecal incontinence, pelvic pain, bowel dysfunction, or sexual dysfunction), obesity, gastroparesis, or psychiatric disorders (e.g., depression, mania, obsessive compulsive disorder, anxiety disorders, and the like). In this manner, system 100 may be configured to provide therapy taking the form of deep brain stimulation (DBS), peripheral nerve stimulation (PNS), peripheral nerve field stimulation (PNFS), cortical stimulation (CS), pelvic floor stimulation, gastrointestinal stimulation, or any other stimulation therapy capable of treating a condition of patient 105.

In some examples, lead 130 may include one or more sensors configured to allow IMD 110 to monitor one or more parameters of patient 105, such as patient activity, pressure, temperature, or other characteristics. Additionally, or alternatively, the one or more sensors may be separate from that of lead 130 (e.g., as a separate sensor communicatively coupled to IMD 110). The one or more sensors may be provided in addition to, or in place of, therapy delivery by lead 130. In some examples, IMD 110 may determine and/or adjust the ratio of non-informing control pulses to informing control pulses for a train of control pulses delivered to the patient based on one or more parameters monitored by the one or more sensors. In some examples, the one or more sensors may include a sensor configured to monitor glutamate levels of patient 105, e.g., so that IMD 110 may control the ratio of cathodic active first phase and anodic active first phase of non-informing control pulses delivered via lead 130, e.g., in a closed-loop feedback manner.

IMD 110 is configured to deliver electrical stimulation therapy to patient 105 via selected combinations of electrodes carried by one or both of leads 130, alone or in combination with an electrode carried by or defined by an outer housing of IMD 110. The target tissue for the electrical stimulation therapy may be any tissue affected by electrical stimulation, which may be in the form of electrical stimulation pulses or continuous waveforms. In some examples, the target tissue includes nerves, smooth muscle or skeletal muscle. In the example illustrated by FIG. 1, the target tissue is tissue proximate spinal cord 120, such as within an intrathecal space or epidural space of spinal cord 120, or, in some examples, adjacent nerves that branch off spinal cord 120. Leads 130 may be introduced into spinal cord 120 in via any suitable region, such as the thoracic, cervical or lumbar regions. Stimulation of spinal cord 120 may, for example, prevent pain signals from traveling through spinal cord 120 and to the brain of patient 105. Patient 105 may perceive the interruption of pain signals as a reduction in pain and, therefore, efficacious therapy results. In other examples, stimulation of spinal cord 120 may produce paresthesia which may be reduce the perception of pain by patient 105, and thus, provide efficacious therapy results.

IMD 110 generates and delivers electrical stimulation therapy to a target stimulation site within patient 105 via the electrodes of leads 130 to patient 105 according to one or more therapy stimulation programs. A therapy stimulation program defines values for one or more parameters that define an aspect of the therapy delivered by IMD 110 according to that program. For example, a therapy stimulation program that controls delivery of stimulation by IMD 110 in the form of pulses may define values for voltage or current pulse amplitude, pulse width, and pulse rate (e.g., pulse frequency) for stimulation pulses delivered by IMD 110 according to that program. A therapy stimulation program may define control pulses and/or informed pulses when these pulses are configured to contribute to the therapeutic effect (e.g., paresthesia, pain blocking, etc.) for patient 105.

Furthermore, IMD 110 is configured to deliver control stimulation (e.g., in the form of a train of control pulses, where the train includes both non-informing and informing control pulses) to patient 105 via a combination of electrodes of leads 130, alone or in combination with an electrode carried by or defined by an outer housing of IMD 110. The tissue targeted by the control stimulation may be the same tissue targeted by the electrical stimulation therapy, but IMD 110 may deliver control pulses via the same, at least some of the same, or different electrodes, and intended to elicit a detectable ECAP signal. This control stimulation may (e.g., therapeutic stimulation) or may not (e.g., non-therapeutic stimulation) contribute to a therapeutic effect for the patient. Since control pulses can be delivered in an interleaved manner with informed pulses, a clinician and/or user may select any desired electrode combination for informed pulses. Like the electrical stimulation therapy, the control stimulation may be in the form of electrical stimulation pulses or continuous waveforms.

In one example, each informing control pulse may include a balanced, bi-phasic square pulse that employs an active recharge phase. For example, each informing control pulse may include a first active phase followed by a second active phase of equal and opposite charge. Conversely, non-informing control pulses may include a monophasic pulse followed by a passive recharge/second phase. For example, the non-informing control pulses may include a first active phase followed by a second passive phase, where each of the first phase and second phase have equal but opposite charges. Although not necessary, the control pulses may include an interphase interval between the positive and negative phase, e.g., to promote propagation of the nerve impulse in response to the first phase of the informing pulse. The control stimulation may be delivered without interrupting the delivery of the electrical stimulation informed pulses, such as during the window between consecutive informed pulses. All or some of the control pulses may elicit an ECAP signal from the tissue. In the case of informing control pulses, IMD 110 may sense the ECAP signal via two or more electrodes on leads 130. In cases where the control pulses are applied to spinal cord 120, the signal may be sensed by IMD 110 from spinal cord 120. As discussed herein, the control stimulation may contribute, alone or in part, to the therapeutic effect received by the patient. In other words, control pulses may be delivered to provide therapy without any additional informed pulses in some examples. In examples in which the control pulses alone can provide therapy to the patient, the control stimulation may be the therapy stimulation for that patient

IMD 110 delivers control stimulation (e.g., in the form of a train of informing control pulses interleaved with non-informing control pulses) to a target stimulation site within patient 105 via the electrodes of leads 130 according to one or more ECAP test stimulation programs. The one or more ECAP test stimulation programs may be stored in a memory of IMD 110. Each ECAP test program of the one or more ECAP test stimulation programs includes values for one or more parameters that define an aspect of the control stimulation delivered by IMD 110 according to that program, such as current or voltage amplitude, pulse width, pulse frequency, electrode combination, and, in some examples timing based on informed pulses to be delivered to patient 105. In some examples, IMD 110 delivers control stimulation to patient 105 according to multiple ECAP test stimulation programs.

A user, such as a clinician or patient 105, may interact with a user interface of an external programmer 150 to program IMD 110. Programming of IMD 110 may refer generally to the generation and transfer of commands, programs, or other information to control the operation of IMD 110. In this manner, IMD 110 may receive the transferred commands and programs from programmer 150 to control electrical stimulation therapy (e.g., informed pulses, and in some examples non-informing and/or informing control pulses) and control stimulation (e.g., control pulses). For example, external programmer 150 may transmit therapy stimulation programs, ECAP test stimulation programs, stimulation parameter adjustments, therapy stimulation program selections, ECAP test program selections, user input, or other information to control the operation of IMD 110, e.g., by wireless telemetry or wired connection.

In some cases, external programmer 150 may be characterized as a physician or clinician programmer if it is primarily intended for use by a physician or clinician. In other cases, external programmer 150 may be characterized as a patient programmer if it is primarily intended for use by a patient. A patient programmer may be generally accessible to patient 105 and, in many cases, may be a portable device that may accompany patient 105 throughout the patient's daily routine. For example, a patient programmer may receive input from patient 105 when the patient wishes to terminate or change electrical stimulation therapy. In general, a physician or clinician programmer may support selection and generation of programs by a clinician for use by IMD 110, whereas a patient programmer may support adjustment and selection of such programs by a patient during ordinary use. In other examples, external programmer 150 may include, or be part of, an external charging device that recharges a power source of IMD 110. In this manner, a user may program and charge IMD 110 using one device, or multiple devices.

As described herein, information may be transmitted between external programmer 150 and IMD 110. Therefore, IMD 110 and programmer 150 may communicate via wireless communication using any techniques known in the art. Examples of communication techniques may include, for example, radiofrequency (RF) telemetry and inductive coupling, but other techniques are also contemplated. In some examples, programmer 150 may include a communication head that may be placed proximate to the patient's body near the IMD 110 implant site to improve the quality or security of communication between IMD 110 and programmer 150. Communication between programmer 150 and IMD 110 may occur during power transmission or separate from power transmission.

In some examples, IMD 110, in response to commands from external programmer 150, delivers electrical stimulation therapy according to a plurality of therapy stimulation programs to a target tissue site of the spinal cord 120 of patient 105 via electrodes (not depicted) on leads 130. In some examples, IMD 110 may modify therapy stimulation programs as therapy needs of patient 105 evolve over time. For example, the modification of the therapy stimulation programs may cause the adjustment of at least one parameter of the plurality of informed pulses. When patient 105 receives the same therapy for an extended period, the efficacy of the therapy may be reduced. In some cases, parameters of the plurality of informed pulses may be automatically updated.

Efficacy of electrical stimulation therapy may be indicated by one or more characteristics (e.g. an amplitude of or between one or more peaks or an area under the curve of one or more peaks) of an action potential that is evoked by a stimulation pulse delivered by IMD 110 (i.e., a characteristic of the ECAP signal). Electrical stimulation therapy delivery by leads 130 of IMD 110 may cause neurons within the target tissue to evoke a compound action potential that travels up and down the target tissue, eventually arriving at sensing electrodes of IMD 110. Furthermore, control stimulation may also elicit at least one ECAP, and ECAPs responsive to control stimulation may also be a surrogate for the effectiveness of the therapy. The amount of action potentials (e.g., number of neurons propagating action potential signals) that are evoked may be based on the various parameters of electrical stimulation pulses such as amplitude, pulse width, frequency, pulse shape (e.g., slew rate at the beginning and/or end of the pulse), etc. The slew rate may define the rate of change of the voltage and/or current amplitude of the pulse at the beginning and/or end of each pulse or each phase within the pulse. For example, a very high slew rate indicates a steep or even near vertical edge of the pulse, and a low slew rate indicates a longer ramp up (or ramp down) in the amplitude of the pulse. In some examples, these parameters may contribute to an intensity of the electrical stimulation. In addition, a characteristic of the ECAP signal (e.g., an amplitude) may change based on the distance between the stimulation electrodes and the nerves subject to the electrical field produced by the delivered control pulses.

In one example, each informed pulse may have a pulse width greater than approximately 300 µs, such as between approximately 300 µs and 1000 µs (i.e., 1 millisecond) in some examples. At these pulse widths, IMD 110 may not sufficiently detect an ECAP signal because the informed pulse is also detected as an artifact that obscures the ECAP signal. If ECAPs are not adequately recorded, then ECAPs arriving at IMD 110 cannot be compared to the target ECAP characteristic (e.g. a target ECAP amplitude), and electrical therapy stimulation cannot be altered according to responsive ECAPs. When informed pulses have these longer pulse widths, IMD 110 may deliver control stimulation in the form of control pulses. The control pulses may have pulse widths of less than approximately 300 µs, such as a bi-phasic informing control pulse with each phase having a duration of approximately 100 µs. Since the control pulses may have shorter pulse widths than the informed pulses, the ECAP signal may be sensed and identified following each control pulse (or only after each informing control pulse) and used to inform IMD 110 about any changes that should be made to the informed pulses (and control pulses in some examples). In some examples, at least some informed pulses may have pulse widths less than approximately 300 µs. In such examples, control pulses interleaved with the informed pulses may have pulse widths shorter than the pulse widths of informed pulses. In other examples, a control pulse may have a pulse width greater than the pulse width of the informed pulse. In general, the term "pulse width" refers to the collective duration of every phase, and interphase interval when appropriate, of a single pulse. A single pulse may include a single active phase in some examples (i.e., a monophasic pulse followed by a passive recharge phase) or two or more active phases in other examples (e.g., a bi-phasic pulse or a tri-phasic pulse). The pulse width defines a period of time beginning with a start time of a first phase of the pulse and concluding with an end time of a last phase of the pulse (e.g., a biphasic pulse having a positive phase lasting 100 µs, a negative phase lasting 100 µs, and an interphase interval lasting 30 µs defines a pulse width of 230 µs).

In some examples, IMD 110 adjusts stimulation parameter values for informed pulses are based on comparing the value of a characteristic of a measured ECAP signal elicited by an informing control pulse to a target ECAP characteristic value. During delivery of control pulses, such as informing control pulses) defined by one or more ECAP test stimulation programs, IMD 110, via two or more electrodes interposed on leads 130, senses electrical potentials of tissue of the spinal cord 120 of patient 105 to measure the electrical activity of the tissue. IMD 110 senses ECAPs from the target tissue of patient 105, e.g., with electrodes on one or more leads 130 and associated sense circuitry. In some examples, IMD 110 receives a signal indicative of the ECAP from one or more sensors, e.g., one or more electrodes and circuitry, internal or external to patient 105. Such an example signal may include a signal indicating an ECAP of the tissue of the patient 105. Examples of the one or more sensors include one or more sensors configured to measure a compound action potential of the patient 105, or a physiological effect indicative of a compound action potential. For example, to measure a physiological effect of a compound action potential, the one or more sensors may be an accelerometer, a pressure sensor, a bending sensor, a sensor configured to detect a posture of patient 105, or a sensor configured to detect a respiratory function of patient 105. However, in other examples, external programmer 150 receives a signal indicating a compound action potential in the target tissue of patient 105 and transmits a notification to IMD 110.

In the example of FIG. 1, IMD 110 described as performing a plurality of processing and computing functions. However, external programmer 150 instead may perform one, several, or all of these functions. In this alternative example, IMD 110 functions to relay sensed signals to external programmer 150 for analysis, and external programmer 150 transmits instructions to IMD 110 to adjust the one or more parameters defining the electrical stimulation therapy based on analysis of the sensed signals. For example, IMD 110 may relay the sensed signal indicative of an ECAP to external programmer 150. External programmer 150 may compare the parameter value of the ECAP to the target ECAP characteristic value, and in response to the comparison, external programmer 150 may instruct IMD 110 to adjust one or more parameter that defines the electrical stimulation informed pulses and, in some examples, control pulses, delivered to patient 105.

The control stimulation parameters (including the ratio of informing to non-informing pulses and the morphology of both the informing to non-informing pulses) and the target ECAP characteristic values may be initially set at the clinic but may be set and/or adjusted at home by patient 105. Once the target ECAP characteristic values are set, the example techniques allow for automatic adjustment of informed pulse parameters to maintain consistent volume of neural activation and consistent perception of therapy for the patient when the electrode-to-neuron distance changes. The ability to change the stimulation parameter values may also allow the therapy to have long term efficacy, with the ability to keep the intensity of the stimulation (e.g., as indicated by the ECAP) consistent by comparing the measured ECAP values to the target ECAP characteristic value. IMD 110 may perform these changes without intervention by a physician or patient 105.

In some examples, the system may change the target ECAP characteristic value over a period of time. The system may be programmed to change the target ECAP characteristic in order to adjust the intensity of informed pulses to provide varying sensations to the patient (e.g., increase or decrease the volume of neural activation). In one example, a system may be programmed to oscillate a target ECAP characteristic value between a maximum target ECAP characteristic value and a minimum target ECAP characteristic value at a predetermined frequency to provide a sensation to the patient that may be perceived as a wave or other sensation that may provide therapeutic relief for the patient. The maximum target ECAP characteristic value, the minimum target ECAP characteristic value, and the predetermined frequency may be stored in the memory of IMD 110 and may be updated in response to a signal from external programmer 150 (e.g., a user request to change the values stored in the memory of IMD 110). In other examples, the target ECAP characteristic value may be programed to steadily increase or steadily decrease to a baseline target ECAP characteristic value over a period of time. In other examples, external programmer 150 may program the target ECAP characteristic value to automatically change over time according to other predetermined functions or patterns. In other words, the target ECAP characteristic value may be programmed to change incrementally by a predetermined amount or predetermined percentage, the predetermined amount or percentage being selected according to a predetermined function (e.g., sinusoid function, ramp function, exponential function, logarithmic function, or the like). Increments in which the target ECAP characteristic value is changed may be changed for every certain number of pulses or a certain unit of time. Although the system may change the target ECAP characteristic value, received ECAP signals may still be used by the system to adjust one or more parameter values of the informed pulses and/or control pulses in order to meet the target ECAP characteristic value.

FIG. 2A is a block diagram of IMD 200. IMD 200 may be an example of IMD 110 of FIG. 1. In the example shown in FIG. 2A, IMD 200 includes processing circuitry 214, memory 215, stimulation generator 211, sensing circuitry 212, telemetry circuitry 213, sensor 216, and power source 219. Each of these circuits may be or include programmable or fixed function circuitry configured to perform the functions attributed to respective circuitry. For example, processing circuitry 214 may include fixed-function or programmable circuitry, stimulation generator 211 may include circuitry configured to generate stimulation signals such as pulses or continuous waveforms on one or more channels, sensing circuitry 212 may include sensing circuitry for sensing signals, and telemetry circuitry 213 may include telemetry circuitry for transmission and reception of signals. Memory 215 may store computer-readable instructions that, when executed by processing circuitry 214, cause IMD 200 to perform various functions. Memory 215 may be a storage device or other non-transitory medium.

In the example shown in FIG. 2A, memory 215 stores therapy stimulation programs 217 and ECAP test stimulation programs 218 in separate memories within memory 215 or separate areas within memory 215. Memory 215 also stores target ECAP feedback rules 221 and patient ECAP characteristics 222. Each stored therapy stimulation program 217 defines values for a set of electrical stimulation parameters (e.g., a parameter set or set of parameter values), such as a stimulation electrode combination, electrode polarity, current or voltage amplitude, pulse width, pulse rate, and pulse shape. Each stored ECAP test stimulation programs 218 defines values for a set of electrical stimulation parameters (e.g., a control stimulation parameter set), such as a stimulation electrode combination, electrode polarity, current or voltage amplitude, pulse width, pulse rate, and pulse shape for each of the non-informing and informing control pulses. ECAP test stimulation programs 218 may also have additional information such as instructions regarding when to deliver control pulses based on the pulse width and/or frequency of the informed pulses defined in therapy stimulation programs 217.

Accordingly, in some examples, stimulation generator 211 generates electrical stimulation signals in accordance with the electrical stimulation parameters noted above. Other ranges of parameter values may also be useful and may depend on the target stimulation site within patient 105. While stimulation pulses are described, stimulation signals may be of any form, such as continuous-time signals (e.g., sine waves) or the like. Switch circuitry 210 may include one or more switch arrays, one or more multiplexers, one or more switches (e.g., a switch matrix or other collection of switches), or other electrical circuitry configured to direct stimulation signals from stimulation generator 211 to one or more of electrodes 232, 234, or directed sensed signals from one or more of electrodes 232, 234 to sensing circuitry 212. In other examples, stimulation generator 211 and/or sensing circuitry 212 may include sensing circuitry to direct signals to and/or from one or more of electrodes 232, 234, which may or may not also include switch circuitry 210.

Processing circuitry 214 may include any one or more of a microprocessor, a controller, a digital signal processor (DSP), an application specific integrated circuit (ASIC), a field-programmable gate array (FPGA), discrete logic circuitry, or any other processing circuitry configured to provide the functions attributed to processing circuitry 214 herein may be embodied as firmware, hardware, software or any combination thereof. Processing circuitry 214 controls stimulation generator 211 to generate stimulation signals according to therapy stimulation programs 217 and ECAP test stimulation programs 218 stored in memory 215 to apply stimulation parameter values specified by one or more of programs, such as amplitude, pulse width, pulse rate, the pulse shape of each of the stimulation signals, the ratio of informing to non-informing control pulses, the interphase interval for informing and non-informing control pulses, and the ratio of anodic first active phase to cathodic active first phase of non-informing control pulses.

In the example shown in FIG. 2A, the set of electrodes 232 includes electrodes 232A, 232B, 232C, and 232D, and the set of electrodes 234 includes electrodes 234A, 234B, 234C, and 234D. In other examples, a single lead may include all eight electrodes 232 and 234 along a single axial length of the lead. Processing circuitry 214 also controls stimulation generator 211 to generate and apply the stimulation signals to selected combinations of electrodes 232, 234. In some examples, stimulation generator 211 includes a switch circuit (instead of, or in addition to, switch circuitry 210) that may couple stimulation signals to selected conductors within leads 230, which, in turn, deliver the stimulation signals across selected electrodes 232, 234. Such a switch circuit may be a switch array, switch matrix, multiplexer, or any other type of switching circuit configured to selectively couple stimulation energy to selected electrodes 232, 234 and to selectively sense bioelectrical neural signals of a spinal cord of the patient (not shown in FIG. 2A) with selected electrodes 232, 234.

In other examples, however, stimulation generator 211 does not include a switch circuit and switch circuitry 212 does not interface between stimulation generator 211 and electrodes 232, 234. In these examples, stimulation generator 211 comprises a plurality of pairs of voltage sources, current sources, voltage sinks, or current sinks connected to each of electrodes 232, 234 such that each pair of electrodes has a unique signal circuit In other words, in these examples, each of electrodes 232, 234 is independently controlled via its own signal circuit (e.g., via a combination of a regulated voltage source and sink or regulated current source and sink), as opposed to switching signals between electrodes 232, 234.

Electrodes 232, 234 on respective leads 230 may be constructed of a variety of different designs. For example, one or both of leads 230 may include one or more electrodes at each longitudinal location along the length of the lead, such as one electrode at different perimeter locations around the perimeter of the lead at each of the locations A, B, C, and D. In one example, the electrodes may be electrically coupled to stimulation generator 211, e.g., via switch circuitry 210 and/or switching circuitry of the stimulation generator 211, via respective wires that are straight or coiled within the housing of the lead and run to a connector at the proximal end of the lead. In another example, each of the electrodes of the lead may be electrodes deposited on a thin film. The thin film may include an electrically conductive trace for each electrode that runs the length of the thin film to a proximal end connector. The thin film may then be wrapped (e.g., a helical wrap) around an internal member to form the lead 230. These and other constructions may be used to create a lead with a complex electrode geometry.

Although sensing circuitry 212 is incorporated into a common housing with stimulation generator 211 and processing circuitry 214 in FIG. 2A, in other examples, sensing circuitry 212 may be in a separate housing from IMD 200 and may communicate with processing circuitry 214 via wired or wireless communication techniques.

In some examples, one or more of electrodes 232 and 234 may be suitable for sensing the ECAPs. For instance, electrodes 232 and 234 may sense the voltage amplitude of a portion of the ECAP signals, where the sensed voltage amplitude is a characteristic the ECAP signal.

Sensor 216 may include one or more sensing elements that sense values of a respective patient parameter. As described, electrodes 232 and 234 may be the electrodes that sense the parameter value of the ECAP elicited by informing control pulses. Sensor 216 may include one or more accelerometers, optical sensors, chemical sensors, temperature sensors, pressure sensors, or any other types of sensors.

Sensor 216 may output patient parameter values that may be used as feedback to control delivery of therapy. For example, sensor 216 may indicate patient activity, and processing circuitry 214 may increase the frequency of control pulses and ECAP sensing in response to detecting increased patient activity. Additionally, or alternatively, sensor 216 may indicate patient activity, and processing circuitry 214 may adjust the ratio of informing to non-informing control pulses (e.g., while maintaining the frequency at which the control pulses are delivered). In one example, processing circuitry 214 may increase the ratio of informing to non-informing control pulses and corresponding ECAP sensing while maintaining the frequency at which the control pulses are delivered in response to a signal from sensor 216 indicating that patient activity has exceeded an activity threshold. Conversely, processing circuitry 214 may decrease the ratio of informing to non-informing control pulses and corresponding ECAP sensing while maintaining the frequency at which the control pulses are delivered in response to detecting decreased patient activity. For example, in response to sensor 216 no longer indicating that the sensed patient activity exceeds a threshold, processing circuitry 214 may decrease the ratio of informing to non-informing control pulses and corresponding ECAP sensing while maintaining the frequency at which the control pulses are delivered. In this manner, processing circuitry 214 may dynamically deliver control pulses and sense ECAP signals based on patient activity to reduce power consumption of the system when the electrode-to-neuron distance is not likely to change and increase system response to ECAP changes when electrode-to-neuron distance is likely to change. The reduction in power consumption may be realized as a result of the non-informing control pulse requiring less power to deliver compared to informing control pulses (e.g., with the non-informing control pulses having a passive recharge/second phase and the informing control pulses having an active recharge/second phase). Similar dynamic adjustments may also be made by processing circuitry 214 to the interphase interval of the non-informing pulses and/or ratio of an anodic first active phase and a cathodic first active phase for the non-informing pulses. As will be described below, in some examples, processing circuitry 214 may dynamically adjust the ratio of an anodic first active phase and a cathodic first active phase for the non-informing pulses of a control pulse train based on a sensed glutamate level of the patient.

IMD 200 may include additional sensors within the housing of IMD 200 and/or coupled via one of leads 130 or other leads. In addition, IMD 200 may receive sensor signals wirelessly from remote sensors via telemetry circuitry 213, for example. In some examples, one or more of these remote sensors may be external to patient (e.g., carried on the external surface of the skin, attached to clothing, or otherwise positioned external to the patient). In some examples, signals from sensor 216 may indicate a position or body state (e.g., sleeping, awake, sitting, standing, or the like), and processing circuitry 214 may select target ECAP characteristic values according to the indicated position or body state.

Telemetry circuitry 213 supports wireless communication between IMD 200 and an external programmer (not shown in FIG. 2A) or another computing device under the control of processing circuitry 214. Processing circuitry 214 of IMD 200 may receive, as updates to programs, values for various stimulation parameters such as amplitude and electrode combination, from the external programmer via telemetry circuitry 213. Updates to the therapy stimulation programs 217 and ECAP test stimulation programs 218 may be stored within memory 215. Telemetry circuitry 213 in IMD 200, as well as telemetry circuits in other devices and systems described herein, such as the external programmer, may accomplish communication by radiofrequency (RF) communication techniques. In addition, telemetry circuitry 213 may communicate with an external medical device programmer (not shown in FIG. 2A) via proximal inductive interaction of IMD 200 with the external programmer. The external programmer may be one example of external programmer 150 of FIG. 1. Accordingly, telemetry circuitry 213 may send information to the external programmer on a continuous basis, at periodic intervals, or upon request from IMD 110 or the external programmer.

Power source 219 delivers operating power to various components of IMD 200. Power source 219 may include a rechargeable or non-rechargeable battery and a power generation circuit to produce the operating power. Recharging may be accomplished through proximal inductive interaction between an external charger and an inductive charging coil within IMD 200. In other examples, traditional primary cell batteries may be used.

According to the techniques of the disclosure, stimulation generator 211 of IMD 200 receives, via telemetry circuitry 213, instructions to deliver electrical stimulation therapy according to therapy stimulation programs 217 to a target tissue site of the spinal cord of the patient via a plurality of electrode combinations of electrodes 232, 234 of leads 230 and/or a housing of IMD 200. Stimulation generator 211 may receive, via telemetry circuitry 213, user instructions to deliver control stimulation to the patient according to ECAP test stimulation programs 218. Each informing pulse of a plurality of control pulses may elicit an ECAP that is sensed by sensing circuitry 212 via some of electrodes 232 and 234. ECAP test stimulation programs 218 may instruct stimulation generator 211 to deliver a plurality of control pulses interleaved with at least some of the plurality of informed pulses. Processing circuitry 214 may receive, via an electrical signal sensed by sensing circuitry 212, information indicative of an ECAP signal (e.g., a numerical value indicating a characteristic of the ECAP in electrical units such as voltage or power) produced in response to the control stimulation. Therapy stimulation programs 217 may be updated according to the ECAPs recorded at sensing circuitry 212 according to the following techniques.

In one example, the plurality of informed pulses each have a pulse width of greater than approximately 300 µs and less than approximately 2000 µs (i.e., 2 milliseconds). In some examples, the informed pulse width is greater than approximately 300 µs and less than approximately 800 µs. In another example, the informed pulse width is greater than approximately 300 µs and less than approximately 500 µs. In one example, informed pulses have a pulse width of approximately 450 µs and a pulse frequency of approximately 50 Hertz. Amplitude (current and/or voltage) for the informed pulses may be between approximately 0.5 mA (or volts) and approximately 10 mA (or volts), although amplitude may be lower or greater in other examples. In some examples, the system may deliver informed pulses from two or more stimulation programs such that the informed pulses from one stimulation program have at least one different parameter value than the informed pulses from another stimulation program.

Each informing control pulse of the plurality of control pulses may have a pulse width of less than approximately 300 µs. In one example, each informing control pulse of the plurality of control pulses may be a bi-phasic pulse with a positive phase having a width of approximately 100 µs, a negative phase having a width of approximately 100 µs, and an interphase interval having a width of approximately 30 µs. In some examples, the positive phase and negative phase may each be 90 µs, 120 µs, 150 µs or 180 µs in other examples. In other examples, the informing control pulses may each have a pulse width of approximately 60 µs or smaller. Due to the relatively long pulse widths of the plurality of informed pulses, sensing circuitry 212 may be incapable of adequately recording an ECAP signals elicited from an informed pulse because the informed pulse itself will occur during the ECAP signal and obscure the ECAP signal. However, stimulation pulses with pulse widths less than approximately 300 microseconds, such as the plurality of informing control pulses, may be suited to elicit an ECAP which can be sensed after the informing control pulse is completed at sensing circuitry 212 via two or more of electrodes 232 and 234.

Each non-informing control pulse of the plurality of control pulses may have a pulse width of approximately 150 microseconds (µs) (e.g., as defined by the phase width of the active first phase). In one example, each non-informing control pulse of the plurality of control pulses may be have an active first phase having a width of approximately 150 µs, a passive second phase having a width of approximately 4 milliseconds (ms), and an interphase interval having a width of approximately 80 µs. As the second passive phase may be set by the time constant formed from the tissue impedance (a parameter that varies with physiologic state) and the electrode blocking capacitors, the phase width may not be precisely set and may be variable in length. The length of the active first phase may be shorter than the passive second phase. The interphase interval of the non-informing pulses may be less than the interphase interval of the informing control pulses, e.g., since the ECAP may not be sensed following the delivery of the non-informing pulses.

As described above, in some examples, the control pulses may fully provide or partially contribute to the therapy received by the patient by reducing or eliminating symptoms and/or a condition of the patient. In other examples, the control pulses may be non-therapeutic pulses in that the control pulses do not contribute to therapy for the patient. The informing control pulses may be substantially the same as the non-informing control pulses. However, the non-informing control pulses may differ in one or more ways compared to that of the informing control pulses, e.g., in order to reduce power consumption resulting from the delivery of the non-informing control pulses compared to the informing control pulses. For example, the non-informing control pulses may have a passive second phase, the non-informing control pulses may have a longer interphase interval between first and second phases, the non-informing control pulses may have lower amplitude, and/or the first phases of the non-informing control pulses may alternate polarity).

Informing control pulses delivered for the purpose of eliciting detectable ECAP signals may have a current amplitude between approximately 6 mA and 12 mA in some examples, but higher or lower amplitudes may be used in other examples. The frequency of the control pulses may be between approximately 50 Hertz and 400 Hertz in some examples. The ratio of informing to non-informing pulses in the train of control pulses may be selected based on the pulse frequency of the informed pulses, e.g., so that one informing control pulse is delivered for each therapeutic pulse or so that one informing control pulse is delivered for every two, three, four, or more than four therapeutic pulses. The pulse frequency may be a single frequency or a varied frequency over time (e.g., the pulse frequency may change over time according to predetermined pattern, formula, or schedule). In some examples, the system may change the pulse frequency based on patient input or a sensed parameter such as patient posture or activity. Such a relationship may be present when the control pulses are fully interleaved (e.g., alternating) with the informed pulses. However, the frequency of the control pulses may be delivered at a higher frequency than then informed pulses when two or more control pulses are delivered between consecutive informed pulses. In other examples, the frequency of the control pulses may be delivered at a lower frequency than the informed pulses when at least some informed pulses are delivered without a control pulse delivered between them. The frequency of the control pulses may be delivered at a frequency that varies over time if the system is configured to adjust control pulse delivery, and the resulting ECAP sensing, based on other factors such as patient activity. In other examples, the frequency of the control pulses may remain substantially constant but the ratio of informing control pulse to non-informing control pulse of the delivered control pulse train may be varied, e.g., based on the frequency of the informed pulses, which may be dynamically varied.

In one example, the predetermined pulse frequency of the plurality of informed pulses may be less than approximately 400 Hertz. In some examples, the predetermined pulse frequency of the plurality of informed pulses may be between approximately 50 Hertz and 70 Hertz. In one example, the predetermined pulse frequency of the plurality of informed pulses may be approximately 60 Hertz. However, the informed pulses may have frequencies greater than 400 Hertz or less than 50 Hertz in other examples. In some examples, the predetermined pulse frequency of the informed pulses may be a single frequency or a frequency that varies over time. In addition, the informed pulses may be delivered in bursts of pulses, with interburst frequencies of the pulses being low enough such that a control pulse and sensed ECAP can still fit within the window between consecutive informed pulses delivered within the burst of informed pulses.

Since each informed pulse of the plurality of informed pulses may be sensed as an artifact that covers, or obscures, the sensing of at least one ECAP, the plurality of control pulses may be delivered to the patient during a plurality of time events. For example, a time event (e.g., a window) of the plurality of time events may be a time (e.g., a window) between consecutive informed pulses of the plurality of informed pulses at the predetermined pulse frequency. One or more informing and/or non-informing control pulses of the plurality of control pulses may be delivered to the patient during each time event. Consequently, the control pulses may be interleaved with at least some of the informed pulses such that the plurality of control pulses are delivered to the patient while informed pulses are not delivered. In one example, an ECAP elicited from to a control pulse delivered during a time event may be recorded by sensing circuitry 212 during the same time event. In another example, two or more ECAPs responsive to two or more respective control pulses delivered during a time event may be recorded by sensing circuitry 212 during the same time event.

In some examples, therapy stimulation programs 217 may be updated according to a plurality of ECAPs received in response to the plurality of control pulses delivered to the patient according to ECAP test stimulation programs 218. For instance, processing circuitry 214 may update therapy stimulation programs 217 in real time by comparing one or more characteristics of ECAPs sensed by sensing circuitry 212 with target ECAP characteristics stored in memory 215 (e.g., patient ECAP characteristics 222). For example, processing circuitry 214 is configured to determine the amplitude of each ECAP signal received at sensing circuitry 212, and processing circuitry 214 is further configured to determine the representative amplitude of at least one respective ECAP signal and compare the representative amplitude of a series of ECAP signals to a target ECAP adjustment window (e.g., the target ECAP amplitude plus and minus a variance which is stored in patient ECAP characteristics 222). Target ECAP adjustment window may thus be a range of amplitudes deviating from target ECAP amplitude. For instance, the target ECAP adjustment window may span from a lower-bound amplitude value (e.g., the target ECAP amplitude minus the variance) to an upper-bound amplitude value (e.g., the target ECAP amplitude plus the variance). Generally, the lower-bound amplitude value is less than the target ECAP amplitude, and the upper-bound amplitude value is greater than target ECAP amplitude.

If the representative amplitude of the at least one respective ECAP signal (e.g., an amplitude of a single ECAP signal or an average of two or more ECAP amplitudes) is greater than the upper-bound amplitude value, processing circuitry 214 may adjust one or more of therapy stimulation programs 217 and ECAP test stimulation programs 218 to decrease the amplitude of informed pulses and control pulses following the at least one respective ECAP. The amplitude of informed pulses and control pulses may be decreased by different predetermined steps or different predetermined percentages. Additionally, if the representative amplitude of the at least one respective ECAP is less than the lower-bound amplitude value, processing circuitry 214 may adjust therapy stimulation programs 217 and ECAP test stimulation programs 218, and the programs 217 and 218 may instruct stimulation generator 211 to increase the amplitude of informed pulses and control pulses following the at least one respective ECAP. Moreover, if the representative amplitude of the at least one respective ECAP is greater than the lower-bound amplitude value and less than the upper-bound amplitude value, processing circuitry 214 may not change programs 217 and 218, and stimulation generator 211 may maintain the amplitude of the informed pulses following the at least one respective ECAP. In one example, adjusting the programs 217 and 218 may include changing one or more parameters of the plurality of informed pulses and the plurality of control pulses. In one example, the at least one respective ECAP may include a series of four consecutive ECAPs.

Processing circuitry 214, in one example, may change the amplitude of the informed pulses and the control pulses following the at least one respective ECAP inversely proportional to the difference between target ECAP amplitude and the representative amplitude of the at least one respective ECAP. For instance, if the representative amplitude of the at least one respective ECAP is 20% lower than target ECAP amplitude, then processing circuitry 214 may update therapy programs 217 and 218 such that the amplitude of informed pulses and the control pulses is increased by 20%. In one example, the representative amplitude may be the mean amplitude of two or more respective ECAP signals sensed by sensing circuitry 212. In other examples, the representative amplitude may be the median amplitude of two or more respective ECAP signal, or a rolling average of two or more respective ECAP signals. In one example, the amplitude of only one of the non-informing and informing control pulses may be adjusted while the other remains the same.

In another example, processing circuitry 214 may determine the amplitude of a respective ECAP signal sensed by sensing circuitry 212. In response to a comparison between the amplitude of the respective ECAP signal and the target ECAP amplitude stored in patient ECAP characteristics 222, processing circuitry 214 may determine a percentage difference between the amplitude of the respective ECAP signal and target ECAP amplitude. Consequently, processing circuitry 214 may adjust the amplitude of subsequent informed pulses to be inversely proportional to the percentage difference between the amplitude of the respective ECAP and target ECAP amplitude.

In other examples, processing circuitry 214 may use the representative amplitude of the at least one respective ECAP to change other parameters of informed pulses to be delivered, such as pulse width, pulse frequency, and pulse shape. All of these parameters may contribute to the intensity of the informed pulses, and changing one or more of these parameter values may effectively adjust the informed pulse intensity to compensate for the changed distance between the stimulation electrodes and the nerves indicated by the representative amplitude of the ECAP signals.

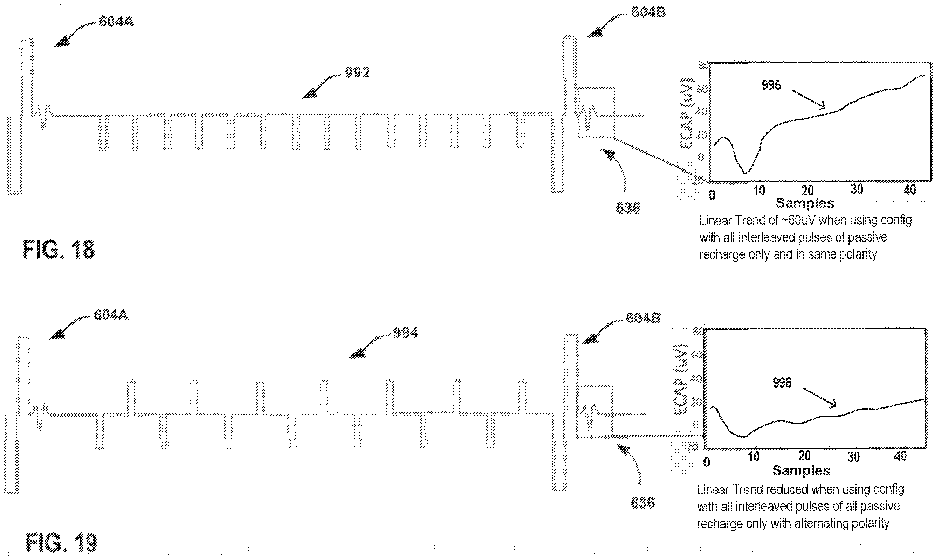

In another example, processing circuitry 214 may sense, via sensing circuitry 212, one or more ECAP signals elicited by the delivery of one or more control pulses and then determine a linear trend for the sensed electrical signal(s). The degree of linear trend may be determined, among other approaches, by estimating the slope of the sensed electrical recording following the evoked potential. In the example of FIG. 18, for instance, an ECAP 636 elicited by control pulse 604B is present from samples 5 to 15, with the samples being measurements acquired serially, e.g., at a frequency of 25 kHz. The slope of the remaining portion of the recording-from samples 15 to 45-is then determined by fitting to an equation of form (ECAP) = (Artifact Slope)(Sample) + (ECAP Intercept). If the calculated artifact slope that represents here the linear trend is an excess of a pre-determined threshold value, an algorithmic choice may be made to take further steps to reduce the trend as described herein, e.g., by delivering a train of pulses with alternating polarity of an active first phase and a second passive phase as shown in FIG. 19. In other instances, the electrical recording may have a curvilinear morphology. Fitting to a more complex function that includes higher order quadratic components may then be necessary to identify the linear trend. Such an approach may be necessary if the curvature of the measured response-as assessed with the second derivative-exceeds a predetermined value. Regardless of the particular technique used to determine the linear trend for sensed elicited electrical signals. Processing circuitry 214 may control one or more parameters of a train of informed pulses based on the determined trend. For example, processing circuitry 214 may compare the determined linear trend to a threshold value and control the informed pulses based on the comparison. A linear trend in ECAP values increasing or decreasing at a rate above a threshold value may be indicative of excessive charge remaining on electrodes prior to sensing ECAP signals. If the determined linear trend is less than or equal to the threshold value, processing circuitry 214 may control stimulation generator 211 to deliver a plurality of informed pulses each having an active first phase of the same polarity, e.g., with a passive or active second phase of the opposite polarity. Conversely, if the determined linear trend is greater than the threshold value, processing circuitry 214 may control stimulation generator 211 to deliver a plurality of informed pulses with alternating polarity first phases and passive second phases. The delivery of the informed pulses in such a manner may reduce the linear trend observed for ECAPs elicited by subsequently delivered informing control pulses, which may facilitate better sensing of ECAPs by IMD 110.

In some examples, leads 230 may be linear 8-electrode leads (not pictured); sensing and stimulation delivery may each be performed using a different set of electrodes. In a linear 8-electrode lead, each electrode may be numbered consecutively from 0 through 7. For instance, a control pulse may be generated using electrode 1 as a cathode and electrodes 0 and 2 as anodes (e.g., a guarded cathode), and a respective ECAP signal may be sensed using electrodes 6 and 7, which are located on the opposite end of the electrode array. This strategy may minimize the interference of the stimulation pulse with the sensing of the respective ECAP. Other electrode combinations may be implemented, and the electrode combinations may be changed using the patient programmer via telemetry circuitry 213. For example, stimulation electrodes and sensing electrodes may be positioned closer together. Shorter pulse widths for the control pulses may allow the sensing electrodes to be closer to the stimulation electrodes.

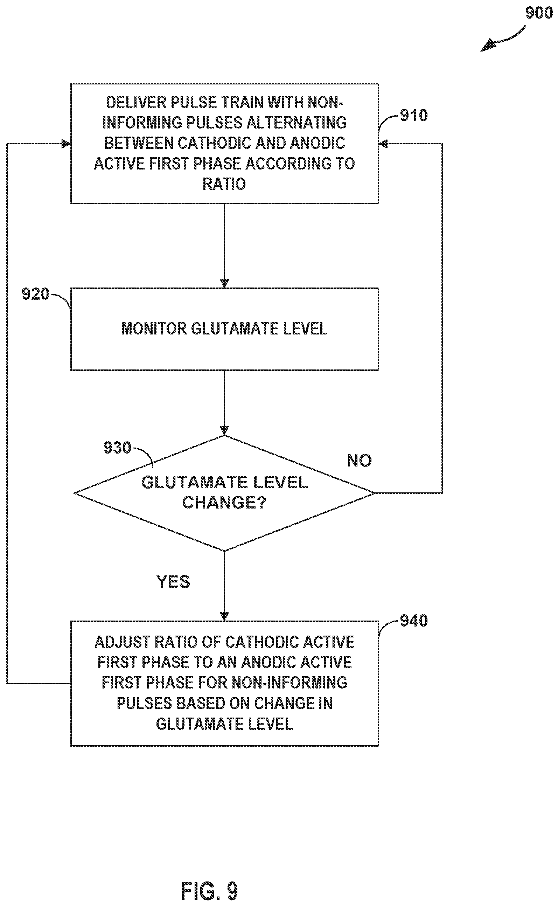

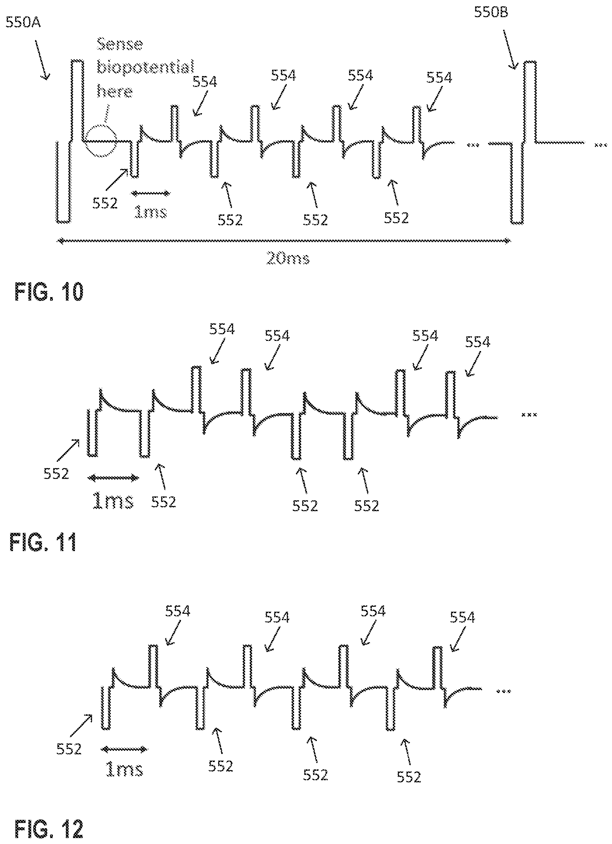

ECAP feedback rules 221 may define how processing circuitry 214 uses the sensed ECAP signals as feedback for changing one or more parameters that define informed pulses and stored as therapy stimulation programs 217. For example, ECAP feedback rules 221 may specify that the percentage difference between the representative ECAP amplitude and the target ECAP amplitude is used to inversely adjust the current amplitude of informed pulses to the same proportion as the percentage difference, such as the technique described in FIG. 9. As another example, ECAP feedback rules 221 may specify that the difference between the target ECAP amplitude is multiplied by a gain value and added to the previous current amplitude of the informed pulses and control pulses as described in FIG. 11. In any case, ECAP feedback rules 221 may instruct processing circuitry 14 how to adjust informed pulses and/or control pulses based on the sensed ECAP signals.

In one example, sensor 216 may detect a change in activity or a change in posture of the patient. Processing circuitry 214 may receive an indication from sensor 216 that the activity level or posture of the patient is changed, and processing circuitry 214 may be configured to initiate or change the delivery of the plurality of control pulses according to the ECAP test stimulation programs 218. For example, processing circuitry 214 may increase the ratio of informing control pulses to non-informing control pulses and respective ECAP sensing after informing control pulses in response to receiving an indication that the patient activity has increased, which may indicate that the distance between electrodes and nerves will likely change. Alternatively, processing circuitry 214 may decrease the ratio of informing control pulses to non-informing control pulses and respective ECAP sensing after informing control pulses in response to receiving an indication that the patient activity has decreased. In some examples, one or more parameters (e.g., frequency, amplitude, slew rate, pulse duration, or the like) may be adjusted (e.g., increased or decreased) in response to receiving an indication that the patient activity has changed. Processing circuitry 214 may be further configured to update therapy stimulation programs 217 and ECAP test stimulation programs 218 according to the signal received from sensor 216.

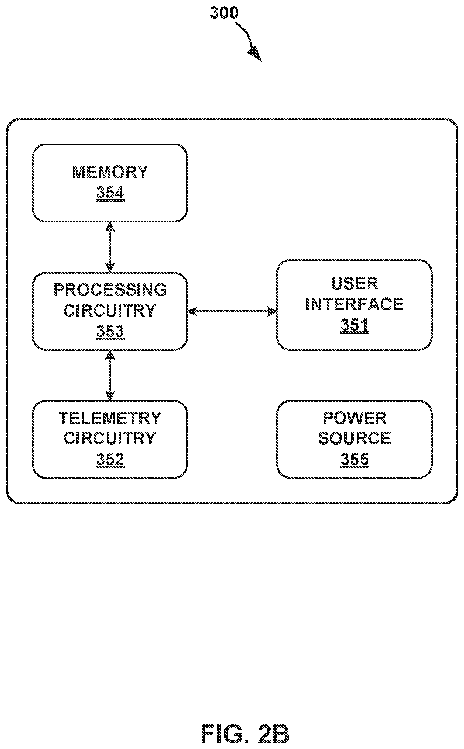

FIG. 2B is a block diagram of the example external programmer 300. External programmer 300 may be an example of external programmer 150 of FIG. 1. Although programmer 300 may generally be described as a hand-held device, programmer 300 may be a larger portable device or a more stationary device. In addition, in other examples, programmer 300 may be included as part of an external charging device or include the functionality of an external charging device. As illustrated in FIG. 2B, programmer 300 may include a processing circuitry 353, memory 354, user interface 351, telemetry circuitry 352, and power source 355. Memory 354 may store instructions that, when executed by processing circuitry 353, cause processing circuitry 353 and external programmer 300 to provide the functionality ascribed to external programmer 300 throughout this disclosure. Each of these components, circuitry, or modules, may include electrical circuitry that is configured to perform some, or all of the functionality described herein. For example, processing circuitry 353 may include processing circuitry configured to perform the processes discussed with respect to processing circuitry 353.

In general, programmer 300 comprises any suitable arrangement of hardware, alone or in combination with software and/or firmware, to perform the techniques attributed to programmer 300, and processing circuitry 353, user interface 351, and telemetry circuitry 352 of programmer 300. In various examples, programmer 300 may include one or more processors, such as one or more microprocessors, DSPs, ASICs, FPGAs, or any other equivalent integrated or discrete logic circuitry, as well as any combinations of such components. Programmer 300 also, in various examples, may include a memory 354, such as RAM, ROM, PROM, EPROM, EEPROM, flash memory, a hard disk, a CD-ROM, comprising executable instructions for causing the one or more processors to perform the actions attributed to them. Moreover, although processing circuitry 353 and telemetry circuitry 352 are described as separate modules, in some examples, processing circuitry 353 and telemetry circuitry 352 are functionally integrated. In some examples, processing circuitry 353 and telemetry circuitry 352 correspond to individual hardware units, such as ASICs, DSPs, FPGAs, or other hardware units.

Memory 354 (e.g., a storage device) may store instructions that, when executed by processing circuitry 353, cause processing circuitry 353 and programmer 300 to provide the functionality ascribed to programmer 300 throughout this disclosure. For example, memory 354 may include instructions that cause processing circuitry 353 to obtain a parameter set from memory, select a spatial electrode movement pattern, or receive a user input and send a corresponding command to IMD 300, or instructions for any other functionality. In addition, memory 354 may include a plurality of programs, where each program includes a parameter set that defines therapy stimulation or control stimulation. Memory 354 may also store data received from a medical device (e.g., IMD 110). For example, memory 354 may store ECAP related data recorded at a sensing module of the medical device, and memory 354 may also store data from one or more sensors of the medical device.

User interface 351 may include a button or keypad, lights, a speaker for voice commands, a display, such as a liquid crystal (LCD), light-emitting diode (LED), or organic light-emitting diode (OLED). In some examples the display may be a touch screen. User interface 351 may be configured to display any information related to the delivery of electrical stimulation, identified patient behaviors, sensed patient parameter values, patient behavior criteria, or any other such information. User interface 351 may also receive user input via user interface 351. The input may be, for example, in the form of pressing a button on a keypad or selecting an icon from a touch screen. The input may request starting or stopping electrical stimulation, the input may request a new spatial electrode movement pattern or a change to an existing spatial electrode movement pattern, of the input may request some other change to the delivery of electrical stimulation.

Telemetry circuitry 352 may support wireless communication between the medical device and programmer 300 under the control of processing circuitry 353. Telemetry circuitry 352 may also be configured to communicate with another computing device via wireless communication techniques, or direct communication through a wired connection. In some examples, telemetry circuitry 352 provides wireless communication via an RF or proximal inductive medium. In some examples, telemetry circuitry 352 includes an antenna, which may take on a variety of forms, such as an internal or external antenna.

Examples of local wireless communication techniques that may be employed to facilitate communication between programmer 300 and IMD 110 include RF communication according to the 802.11 or Bluetooth specification sets or other standard or proprietary telemetry protocols. In this manner, other external devices may be capable of communicating with programmer 300 without needing to establish a secure wireless connection. As described herein, telemetry circuitry 352 may be configured to transmit a spatial electrode movement pattern or other stimulation parameter values to IMD 110 for delivery of electrical stimulation therapy.

In some examples, selection of parameters or therapy stimulation programs may be transmitted to the medical device for delivery to the patient. In other examples, the therapy may include medication, activities, or other instructions that the patient must perform themselves or a caregiver perform for the patient. In some examples, programmer 300 may provide visual, audible, and/or tactile notifications that indicate there are new instructions. Programmer 300 may require receiving user input acknowledging that the instructions have been completed in some examples.

User interface 351 of external programmer 300 may receive an indication from a clinician instructing a processor of the medical device to update one or more therapy stimulation programs or to update one or more ECAP test stimulation programs. Updating therapy stimulation programs and ECAP test stimulation programs may include changing one or more parameters of the stimulation pulses delivered by the medical device according to the programs, such as amplitude, pulse width, frequency, and pulse shape of the informed pulses and/or control pulses. User interface 351 may also receive instructions from the clinician commanding any electrical stimulation, including therapy stimulation and control stimulation to commence or to cease.

The architecture of programmer 300 illustrated in FIG. 2B is shown as an example. The techniques as set forth in this disclosure may be implemented in the example programmer 300 of FIG. 2B, as well as other types of systems not described specifically herein. Nothing in this disclosure should be construed so as to limit the techniques of this disclosure to the example architecture illustrated by FIG. 2B.

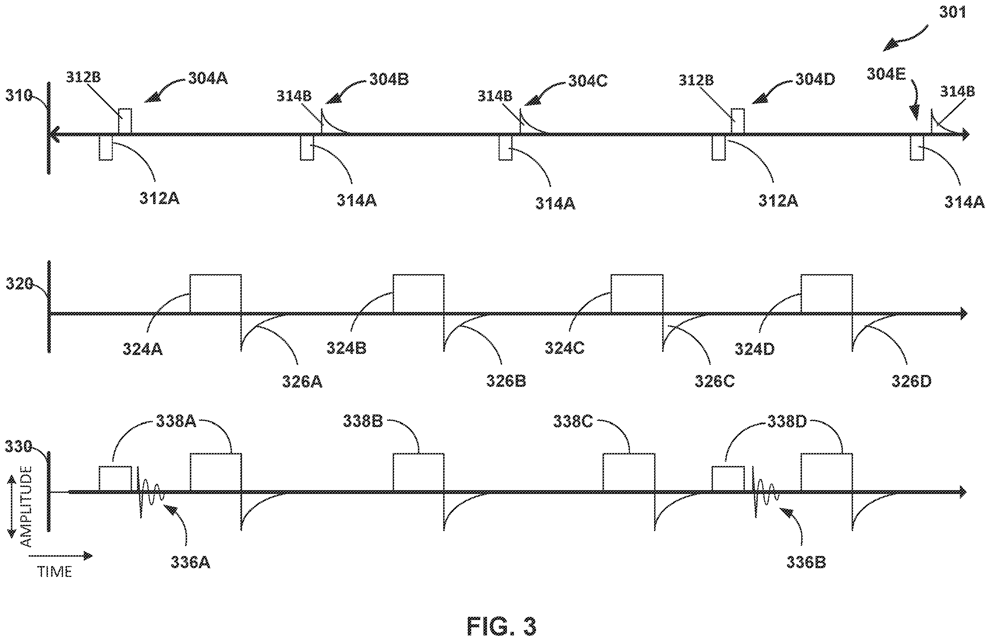

FIG. 3 is a timing diagram 301 illustrating one example of electrical stimulation pulses and respective sensed ECAPs according to some techniques of the disclosure. For convenience, FIG. 3 is described with reference to IMD 200 of FIG. 2A. As illustrated, timing diagram 301 includes first channel 310, a plurality of control pulses 304A-304E (collectively "control pulses 304"), second channel 320, a plurality of informed pulses 324A-324D (collectively "informed pulses 324") including passive recharge phases 326A-326D (collectively "passive recharge phases 326"), third channel 330, a plurality of respective ECAPs 336A and 336B (collectively "ECAPs 336"), and a plurality of stimulation interference signals 338A-338D (collectively "stimulation interference signals 338").

First channel 310 is a time/voltage (and/or current) graph indicating the voltage (or current) of at least one electrode of electrodes 232, 234. In one example, the stimulation electrodes of first channel 310 may be located on the opposite side of the lead as the sensing electrodes of third channel 330. Control pulses 304 may be electrical pulses delivered to the spinal cord of the patient by at least one of electrodes 232, 234.

Control pulses 304 include both informing control pulses 304A and 304D, and non-informing control pulses 304B, 304C, and 304E. Informing control pulses 304A and 304D may be balanced biphasic square pulses with an interphase interval. In other words, each of informing control pulses 304A and 304D are shown with active first phase 312A and an active second phase 312B separated by an interphase interval (not labelled). For both of controls pulses 304A and 304D, the first phase 312A and second phase 312B have equal but opposite charges such that the remaining charge after the respective pulse is delivered is approximately zero. For example, informing control pulse 304A may have a negative voltage (e.g., cathodic voltage) for the same amount of time and amplitude that it has a positive voltage (e.g., anodic voltage). It is noted that the negative voltage phase may be before or after the positive voltage phase.

Conversely, non-informing control pulses 304B, 304C, and 304E may be balanced monophasic square pulses with an interphase interval and a passive recharge phase. In other words, each of non-informing control pulses 304B, 304C, and 304E are shown with active first phase 314A and a passive second phase 314B separated by an interphase interval (not labelled). For each control pulses 304B, 304C, and 304E, the first phase 314A and second phase 314B have equal but opposite charges such that the remaining charge after the respective pulse is delivered is approximately zero. For example, active first phase 314A may have a negative voltage for the same amount of time and amplitude as the positive voltage of the passive second phase 314B. It is noted that first active phase 312A may be a negative voltage phase or a positive voltage phase.

Unlike the informing pulses configured for active recharge, wherein remaining charge on the tissue following a stimulation pulse is instantly removed from the tissue by an opposite applied charge, passive recharge allows tissue to naturally discharge to some reference voltage (e.g., ground or a rail voltage) following the termination of the informed pulse. In some examples, the electrodes of the medical device may be grounded at the medical device body. In this case, following the termination of non-informing pulses 304B, 304C, and 304E, the charge on the tissue surrounding the electrodes may dissipate to the medical device, creating a rapid decay of the remaining charge at the tissue following the termination of the pulse. This rapid decay is illustrated in passive recharge phases 314B. Passive recharge phase 314B may have a duration in addition to the pulse width of the preceding active first phase 314A.

In the example of FIG. 3, active first phase 314A is the same polarity for each of non-informing control pulses 304B, 304C, and 304E. In other examples, the polarity of first active phase 314A may be different for non-informing control pulses 304B, 304C, and 304E (e.g., with first phase 314A of non-informing control pulses 304B, 304E being positive and first phase 314A of non-informing control pulse 304C being negative). The ratio of non-informing control pulses having one polarity (e.g., cathodic) first active phase to non-informing control pulse having the opposite polarity (e.g., anodic) first active phase may vary (e.g., based on a sensed parameter such as glutamate level of the patient) or may be substantially constant.

Control pulses 312 may be delivered according to ECAP test stimulation programs 218 stored in memory 250 of IMD 200, and ECAP test stimulation programs 218 may be updated according to user input via an external programmer and/or may be updated according to a signal from sensor 216. In one example, informing control pulses 304A, 304D may have a pulse width of less than approximately 300 microseconds (e.g., the total time of the positive phase, the negative phase, and the interphase interval is less than 300 microseconds). In another example, informing control pulses 304A, 304D may have a pulse width of approximately 150 µs for each phase of the bi-phasic pulse.

The pulse width of informing pulses 304A, 304D may be different than non-informing pulses 304B, 304C, and 304E. Additionally, or alternatively, the phase width of the first phase of informing pulses 304A, 304D may be different than the first phase of non-informing pulses 304B, 304C, and 304E. Additionally, or alternatively, the phase width of the active second phase of informing pulses 304A, 304D may be different than the passive second phase of non-informing pulses 304B, 304C, and 304E (e.g., where the length of the passive second phase is a decaying exponential set by the tissue impedance and the blocking capacitors. In some examples, the total length of time of first phase 312A, second phase 312B plus any intervening interphase interval between the first and second phase of informing pulse 304A or 304D may be less than the total length of time of the first phase 314A, passive second phase 314B plus any intervening interphase interval between the first and second phase of non-informing pulse 304B, 304C, or 304E. In some examples, the pulse width of active first phase 314A may be up to 300 µs with an interphase interval of up to 150 µs before the start of passive second phase 314B.

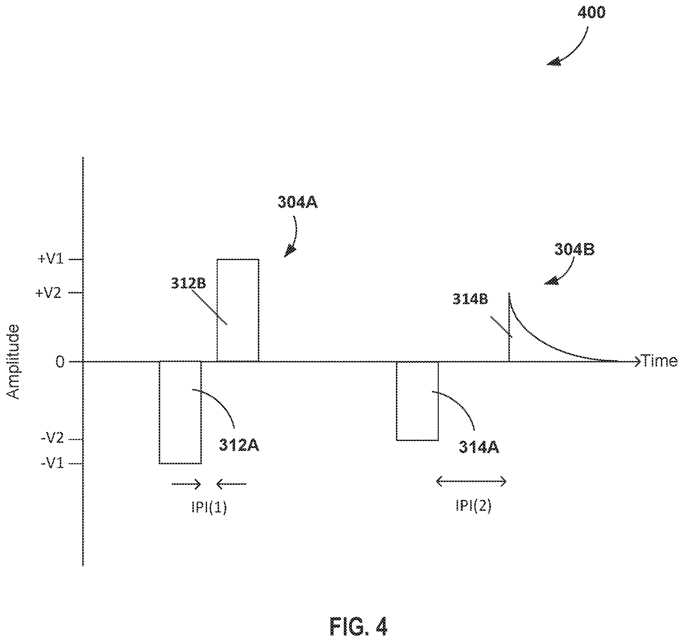

As will be described further below with regard to FIG. 4, the interphase interval for informing pulses 304A, 304D may be different than the interphase interval of the non-informing pulses 304B, 304C, and 304E. For example, the interphase interval for informing pulses 304A, 304D may be less than the interphase interval of the non-informing pulses 304B, 304C, and 304E. Additionally, or alternatively, the amplitude of the first and second active phases 312A and 312B of informing pulses 304A, 304D may be different than the amplitude of the active first phase 314A and passive second phase 314B of the non-informing pulses 304B, 304C, and 304E. For example, the amplitude of the first and second active phases 312A and 312B of informing pulses 304A, 304D may be greater than the amplitude of the active first phase 314A and passive second phase 314B of the non-informing pulses 304B, 304C, and 304E.

As illustrated in FIG. 3, informing control pulses 304A, 304D may be delivered via one or more electrodes that deliver or sense signals corresponding to channel 310. Delivery of control pulses 312 may be delivered by leads 230 in a guarded cathode electrode combination. For example, if leads 230 are linear 8-electrode leads, a guarded cathode combination is a central cathodic electrode with anodic electrodes immediately adjacent to the cathodic electrode.

Informing control pulses 304A, 304D may be delivered on an interleaved basis with non-informing pulses 304B, 304C, and 304E. In the example of FIG. 3, control pulses 304 are delivered according to a repeating pattern of one informed pulse followed by two non-informed pulse. Although not shown in FIG. 3, a singled non-informed pulse would follow non-informed pulse 304E, followed by a single informed pulse, then two non-informed pulses, and so forth, according to such a pattern. The ratio of informed to non-informed pulse for such a pattern would be referred to as a 1:2 ratio. Control pulses 304 may be delivered according to any desired pattern and ratio, and the pattern and/or ratio may be adjusted over a period of time (e.g., based on a sensed parameter). In some examples, the adjustment to the pattern and/or ratio may be made over the period of time while keeping the frequency at which control pulses 304 are delivered substantially constant. A patient's perception of the stimulation therapy may be unchanged by keeping the frequency at which control pulses 304 are delivered constant.

Second channel 320 is a time/voltage (and/or current) graph indicating the voltage (or current) of at least one electrode of electrodes 232, 234 for the informed pulses 324. In one example, the electrodes of second channel 420 may partially or fully share common electrodes with the electrodes of first channel 310 and third channel 330. Informed pulses 324 may also be delivered by the same leads 230 that are configured to deliver control pulses 304. Informed pulses 324 may be interleaved with control pulses 304, such that the two types of pulses are not delivered during overlapping periods of time. However, informed pulses 324 may or may not be delivered by exactly the same electrodes that deliver control pulses 304. Informed pulses 324 may be monophasic pulses with pulse widths of greater than approximately 300 µs and less than approximately 1000 µs, e.g., where the pulse width is the duration of the first phase. In fact, informed pulses 324 may be configured to have longer pulse widths than control pulses 304. As illustrated in FIG. 3, informed pulses 324 may be delivered on channel 420. In some examples, informed pulses 324 may be a burst of interleaved stimulation. For instance, the control pulse 304 may have a first phase width of 150 µs but there may be a burst of 10 pulses with a pulse width of 90 µs delivered at 1 kHz in between the control pulses.

Informed pulses 324 may be configured for passive recharge. For example, each informed pulse 324 may be followed by a passive recharge phase 326 (individually passive recharge phases 326A-326D) to equalize charge on the stimulation electrodes. Unlike a pulse configured for active recharge, wherein remaining charge on the tissue following a stimulation pulse is instantly removed from the tissue by an opposite applied charge, passive recharge allows tissue to naturally discharge to some reference voltage (e.g., ground or a rail voltage) following the termination of the informed pulse. In some examples, the electrodes of the medical device may be grounded at the medical device body. In this case, following the termination of informed pulse 324, the charge on the tissue surrounding the electrodes may dissipate to the medical device, creating a rapid decay of the remaining charge at the tissue following the termination of the pulse. This rapid decay is illustrated in passive recharge phases 326. Passive recharge phase 326 may have a duration in addition to the pulse width of the preceding informed pulse 324. In other examples (not pictured in FIG. 3), informed pulses 324 may be bi-phasic pulses having a positive and negative phase (and, in some examples, an interphase interval between each phase) which may be referred to as pulses including active recharge. An informed pulse that is a bi-phasic pulse may or may not have a following passive recharge phase. Informed pulses 324 may be defined, and be a part of, one or more stimulation programs. Although each of informed pulses 324 are illustrated as having the same parameter values (e.g., the same pulse width, amplitude, and pulse shape), some of informed pulses 324 may have one or more parameters that have different values from each other.

Although informed pulses 324 are shown in FIG. 3 as each including first active phases 324A-324D with the same polarity and second passive phases 326A-326D with the same polarity, as will be described below, e.g., at FIGS. 10-15, in some examples, IMD 110 may deliver a train of pulses such as informed pulses 324 before and/or after the sensing of an electrical signal such as ECAP 336A such that the train includes one or more first pulses having an anodic active first phase and passive second phase of opposite polarity at least partially interleaved with one or more second pulses having a cathodic active first phase and a passive second phase of opposite polarity. Such a stimulation pattern may limit stimulation artifacts and maintain electrochemical balance (e.g., at the tissue site at which the stimulation is delivered to the patient), without the battery capacity draw associated with a train of pulses having an active second (recharge) phase.

Third channel 330 is a time/voltage (and/or current) graph indicating the voltage (or current) of at least one electrode of electrodes 232, 234. In one example, the electrodes of third channel 330 may be located on the opposite side of the lead as the electrodes of first channel 310. ECAPs 336A and 336B may be sensed at electrodes 232, 234 from the spinal cord of the patient in response to informing control pulses 304A and 304D, respectively. ECAPs 336A and 336B are electrical signals which may propagate along a nerve away from the origination of informing control pulses 304A and 304D. In one example, ECAPs 336A and 336B are sensed by different electrodes than the electrodes used to deliver informing control pulses 304A and 304D. As illustrated in FIG. 3, ECAPs 336A and 336B may be recorded on third channel 330.

Stimulation interference signals 338A-338D (e.g., the artifact of the stimulation pulses) may be sensed by leads 230 and may be sensed during the same period of time as the delivery of control pulses 304 and informed pulses 324. Since the interference signals may have a greater amplitude and intensity than ECAPs 336, any ECAPs arriving at IMD 200 during the occurrence of stimulation interference signals 338 may not be adequately sensed by sensing circuitry 212 of IMD 200. However, ECAPs 336 may be sufficiently sensed by sensing circuitry 212 because each ECAP 336 falls after the completion of each a control pulse 304 and before the delivery of the next informed pulse 324. As illustrated in FIG. 3, stimulation interference signals 338 and ECAPs 336 may be recorded on channel 330.

In the example of FIG. 3, pulses 324 may be controlled based the ECAPs 336 elicited by informing control pulses 304A and 304D in the manner described herein. For example, stimulation pulses 324A-324C may be controlled based on ECAP 336A elicited by informing control pulse 304A and sensed by third channel 330, and stimulation pulse 324D may be controlled based on ECAP 336B elicited by informing control pulse 304D and sensed by third channel 330. In such an example, stimulation pulses 324A-324C may be controlled to be substantially the same as each other (e.g., having the same pulse width, amplitude, and the like), and stimulation pulse 324D along with the next two subsequent stimulation pulses (not shown) may be controlled to be substantially the same as each other (e.g., having the same pulse width, amplitude, and the like).

In FIG. 3, ECAPs are not shown as being sensed by IMD 200 directly following the delivery of non-informing pulses 304B, 304C, and 304E. For example, the non-informing pulses 304B, 304C, and 304E may elicit ECAPs although the ECAPs may not be sensed by IMD 200, e.g., because of slewing associated with the passive second phases 314B, or IMD 200 may sense the ECAPs but not adjust or otherwise control the stimulation pulse deliver directly following the sense ECAP.

While the example of FIG. 3 illustrates control pulses 304 being interleaved with informed pulses 324 such that a single informed pulse is delivered between each individual control pulse 304, in other examples, IMD 200 may deliver the interleaved control and informed pulses such that more than one informed pulse may be delivered after an individual control pulse. For example, two or more informed pulses may be delivered after the delivery of control pulse 304A and before the delivery of control pulse 304B, then two or more informed pulses may be delivered after the delivery of control pulse 304B and before the delivery of control pulse 304C, and so forth. As described herein, the amplitude (and/or other stimulation parameters) of the informed pulses may be adjusted given the characteristics of the ECAP evoked from the stimulation complex with the active anodic phase of the preceding informing control pulse.

FIG. 4 is a timing diagram 400 illustrating an example of a plurality of control pulses that may be delivered to a patient via a medical device according to some techniques of the disclosure. For convenience, FIG. 3 is described with reference to IMD 200 of FIG. 2A. As shown, timing diagram 400 represents the delivery of control pulse 304A and control pulse 304B. Control pulse 304A and control pulse 304B may be representative of those similarly numbered controls in the timing diagram 301 of FIG. 3. Furthermore, control pulse 304A may be representative of all the other informing control pulse (e.g., control pulse 304D) in the pulse train represented by timing diagram 301 and, likewise, control pulse 304B may be representative of all the other non-informing control pulses (e.g., control pulses 304C and 304E) in the pulse train represented by timing diagram 301.

As described previously, control pulse 304A may be an informing control pulse and control pulse 304B may be a non-informing control pulse, e.g., based on IMD 200 sensing ECAP 336A elicited by informing control pulse 304A and controlling the delivery of informed pulses 324A-324C based on the sense ECAP and, conversely, IMD 200 not sensing an ECAP elicited by non-informing control pulse 304B and/or not controlling the delivery of one or more informed pulses based on an ECAP.

As shown in FIG. 4, the interphase interval and/or amplitude of informing pulse 304A may be different than the amplitude (e.g., current amplitude or voltage amplitude) of non-informing pulse 304B. For example, the interphase interval IPI(1) of informing control pulse 304A may be less than the interphase interval IPI(2) of non-informing pulse 304B. As described herein, in some examples, it may be beneficial for the non-informing control pulses, such as control pulse 304B, in a train of control pulses to have an interphase interval that is greater than that of the informing control pulses, such as control pulse 304A, in the train of control pulses. For example, longer interphase intervals may enable the use of lower amplitudes of the first phase of the second pulse and less power consumption.

IPI(1) and IPI(2) may be any suitable value. For example, IPI(1) may be about 10 us to about 200 µs, such as about 20 µs to about 100 µs. IPI(2) may be about 20 µs to about 300 µs, such as about 30 µs to about 150 µs. In some examples, IPI (2) may be at least 200% that of IPI(1), such as about 100% to about 400% of IPI(1). In other examples, IPI(1) for the informing control pulses and IPI(2) for the non-informing control pulses may be approximately equal to each other. In some examples, IPI(1) for the informing control pulses in a pulse train and/or IPI(2) for the non-informing control pulses in a pulse train may be dynamically adjusted by IMD 200, e.g., based on the state of charge for power source 219, with power consumption reducing adjustments being made when as the state of charge of power source 219 decreases.

As another example, active first phase 312A of pulse 304A and active second phase 312A have an amplitude of V1 (with V referring to a current or voltage amplitude), with the amplitude of active first phase 312A being (-)V1 and the amplitude of active second phase 312B being (+)V1). Conversely, active first phase 314A of non-informing pulse 304B has an amplitude of (-)V2 and passive second phase 314B has an amplitude of (+)V2. As indicated in FIG. 4, the amplitude of V1 is greater than the amplitude of V2. As described herein, in some examples, it may be beneficial for the non-informing control pulses, such as control pulse 304B, in a train of control pulses to have an amplitude that is less than that of the informing control pulses, such as control pulse 304A, in the train of control pulses. For example, a benefit of examples of the disclosure may be reduced power consumption associated with the delivery of the control pulses as compared to stimulation in which only informing control pulses are delivered. In some examples, the combination of a lower amplitude with potentially an increased IPI for non-informing control pulses may lower the drain on power source 219.

V1 and V2 may be any suitable value. In some examples, V2 may be about 100% or less than that of V1, such as about 70% to about 100% of V1. In other examples, V1 for the informing control pulses and V2 for the non-informing control pulses may be approximately equal to each other or V1 for the informing control pulses may be less than V2 for the non-informing control pulses. In some examples, V1 for the informing control pulses in a pulse train and/or V2 for the non-informing control pulses in a pulse train may be dynamically adjusted by IMD 200, e.g., based on the state of charge for power source 219, with power consumption reducing adjustments being made when as the state of charge of power source 219 decreases.

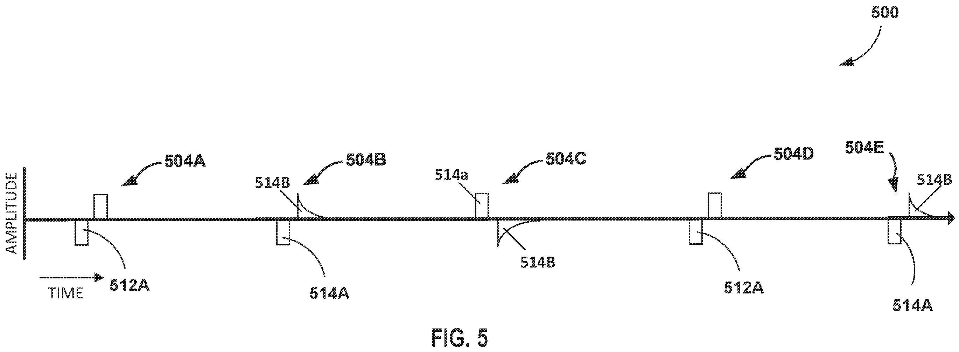

In some examples, the polarity of the active first phase of each non-informing pulses of a train of control pulses may be the same, such as the example of non-informing control pulses 304B, 304C, and 304E shown in FIG. 3. Conversely, FIG. 5 is a timing diagram 500 showing an alternate example of a train of control pulses 504A-504E (collectively control pulses 504) including informing control pulses 504A and 504D as well as non-informing control pulses 504B, 504C, and 504E. The train of control pulses 504 may be delivered in conjunction with informed pulses, e.g., such as informed pulses 324A-324D in the manner shown in FIG. 3.

Informing control pulses 504A and 504E may be the same as that described for similarly numbered informing control pulses 304A and 304D of FIG. 3. Non-informing control pulses 504B, 504C, and 504E may be the same as that described for similarly numbered non-informing control pulses 304B, 304C, and 304E of FIG. 3. However, in the example of FIG. 5, non-informing control pulses 504B, 504C, and 504E between a cathodic active first phase and an anodic active first phase according to a ratio, as compared to non-informing control pulses 304B, 304C, and 304E which all have the same polarity for active first phase 314A.

In an example in which the non-informing control pulses are delivered so that the pulses alternate between a cathodic active first phase and an anodic active first phase, any suitable patter may be used. In the example of FIG. 5, non-informing pulses 504B, 504C, and 504E alternate on a pulse-by-pulse basis between a cathodic active first phase and an anodic active first phase. In such an example, the ratio of non-informing pulse with a cathodic active first phase to non-informing pulse with an anodic active first phase is 1:1. In other examples, the alternating pattern may be selected to provide a desired ratio or vice versa. In some examples, the ratio of non-informing pulse with a cathodic active first phase to non-informing pulse with an anodic active first phase may range from 1 to 10.

In some examples, alternating between a cathodic active first phase and an anodic active first phase may result in different physiological effects on a patient as compared to a case in which all the non-informing pulses in a train of control pulses have the same active first phase (e.g., all cathodic active first phase or all anodic active first phase). For example, benefits of alternating polarity may include a wider field over where the stimulation is delivered (e.g., the activation of more anatomical structures implicated in a positive therapeutic outcome), or less opportunity for habituation to the stimulation. In some examples, delivery of the same polarity results in a less therapeutically optimal neurochemical process. It has been determined both in vitro and in vivo, that the balancing of active phase by an asymmetric or symmetric recharge balance, or the effects of purely cathodic versus purely anodic phase may have a dramatic effect on pain related gene expression. Examples of the disclosure may employ such techniques in order to realize such an example benefit.

As will be describe further below, in some examples, the ratio of non-informing pulse with a cathodic active first phase to non-informing pulse with an anodic active first phase may be dynamically adjusted. For example, IMD 200 may sense glutamate levels for a patient via sensor 216 and adjust the ratio of non-informing pulse with a cathodic active first phase to non-informing pulse with an anodic active first phase, e.g., by adjusting the ratio to be at or near 1:1 when sensed glutamate levels increase above a preprogrammed threshold.

FIGS. 6A-6C are schematic circuit diagrams illustrating the delivery of an active first phase (FIG. 6A) followed by either an active second phase (FIG. 6B) or passive second phase (FIG. 6C). The circuit diagrams are merely examples of circuits that may be employed by IMD 200 for delivering informing control pulses, such as pulses 304A and 304D, and non-informing control pulses, such as pulses 304B, 304C, and 304E, in the manner described herein.

As described herein, a pulse train may include biphasic stimulation pulses (e.g., as informing control pulses) and/or monophasic stimulation (e.g., as non-informing control pulses). In some examples, electrical pulses delivered to chronically activate neural tissue may be biphasic, e.g., with biphasic stimuli preferred over monophasic stimulation for various considerations. The first phase of stimulation of the biphasic stimulation pair may serve to activate the tissue at the working electrode, while the second phase serves to reverse undesirable electrochemistry or deleterious reaction products which formed during the first phase.

By means of an example, the example circuits of FIGS. 6A-6C are constructed to generate regulated current through tissue. Each electrode E1, E2 is connected to a "slice" which consists of a constant current sink/source pair and a switch to ground, via a blocking capacitor. The blocking capacitor serves to isolate the tissue from DC currents should a fault occur in the electronics, and it has utility in generating the second phase of the bipolar stimulus. During the active first phase, current is sourced from VSUPPLY through E2 and is sunk at E1. E1 acts as the cathode and E2 acts as the anode. During the second phase, current is sourced from VSUPPLY through E2 and is sunk at E1. In this second phase, E1 acts as the anode and E2 acts as the cathode. When the current for the second phase is supplied from VSUPPLY, it is referred to as active anodic stimulation (e.g., as reflected in FIG. 6B). As small mismatches exist between the current sourcing/sinking capability of each slice, the switches to ground are closed at the end of the biphasic stimulus to neutralize any charge which has developed on the DC blocking capacitors.

The second phase of stimulation may also be achieved exclusively using the switches to ground; in the example of FIG. 6A and 6C, for instance, current may not need to be actively injected from E1 into E2. After the first phase of stimulation is delivered, a voltage is developed across the DC blocking capacitors. The switches to ground are then closed. While these switches are shown as connected to "ground," any other convenient potential may be employed. The charge developed across the capacitors discharges passively in an exponential fashion, with a time constant set by the series combination of the capacitors and the tissue resistance. This may be referred to as passive anodic stimulation and is illustrated in FIG. 6C.

Passive anodic stimulation may be desirable for IMDs because it does not draw current from VSUPPLY, a concern for devices powered by batteries. However, passive recharge may be problematic for applications where microvolt-level biopotential sensing, such as an ECAP or LFP, is desired shortly after delivery of the stimulation. The long-tailed slewing associated with the passive anodic phase represents an in-band aggressor for the biopotential.

As described herein, a train of pulses in which at least some of the pulses are biphasic pulses that facilitate sensing elicited electrical signals, such as the informing control pulses described herein, are interleaved with monophasic pulses, such as the non-informing control pulses described herein. In some examples, the pulse train may be a hybrid of such pulses where passive anodic stimulation pulses are time-interleaved with active anodic stimulation pulses. Such an example pulse train may allow for more current draw savings than a fully active anodic pulse train, but with more capability for bipotential sensing than a fully passive anodic pulse train. In an exemplary spinal cord stimulation where 50 Hz pulse train is intended, for instance, every other pulse might employ active recharge (e.g., by alternating between a single informing control pulse and a single non-informing control pulse). Such an example may be preferred to delivering a pulse train with active recharge at 25 Hz alone as it may not result in an appropriate therapeutic outcome for the patient, e.g., because of perceived changes by the patient as described herein.

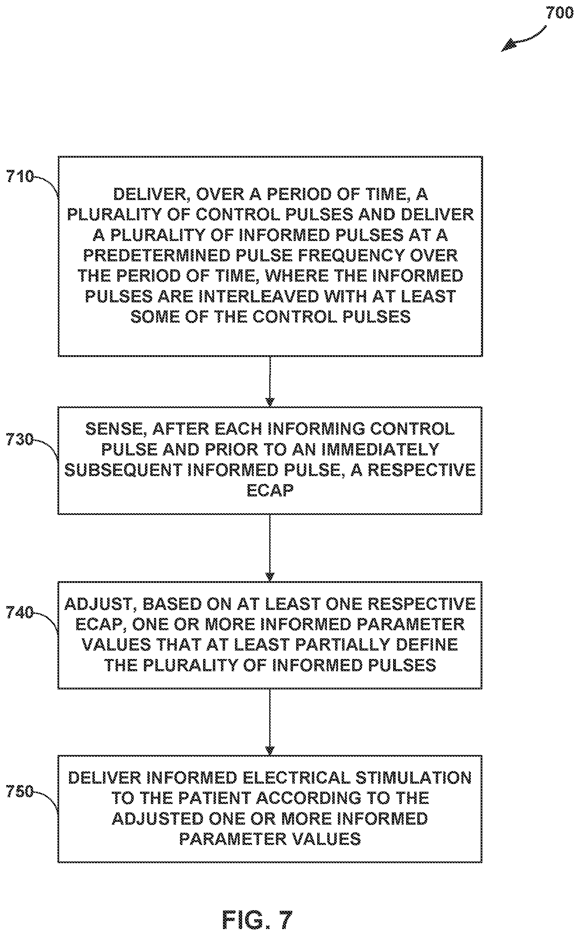

FIG. 7 is a flowchart illustrating an example operation 700 for therapy delivery according to the techniques of this disclosure. For convenience, FIG. 7 is described with respect to IMD 110 of FIG. 1. However, the techniques of FIG. 7 may be performed by different components of IMD 110 or by additional or alternative medical devices. The example of FIG. 7 may be employed to deliver the example electrical stimulation represented by the timing diagram 301 of FIG. 3.

In the example of FIG. 7, IMD 110 may deliver electrical stimulation therapy to patient 105, the electrical stimulation therapy comprising a plurality of control pulses delivered at a predetermined pulse frequency over a period of time and also a plurality of informed pulses delivered at a predetermined pulse frequency over the period of time, where the informed pulses are at least partially interleaved with the control pulses (710). As described herein, the control pulses may include informing control pulses having an active first phase and an active second phase, as well as non-informing pulse having an active first phase and a passive second phase. Additionally, or alternatively, the active first phase of the non-informing control pulses may alternate polarity as described herein. Additionally, or alternatively, the non-informing control pulses may have a longer interphase interval and/or lower amplitude compared that of the informing control pulses. In some examples, one or more control pulses may be delivered between consecutive informed pulses. As another example, one or more informed pulses may be delivered between consecutive control pulses.

IMD 110 may sense, after an informing control pulse of the plurality of control pulses and prior to an immediately subsequent informed pulse of the plurality of informed pulses, a respective ECAP (730). Subsequent to the sensing, IMD 110 may adjust, based on at least one respective ECAP, one or more parameter values that at least partially define the plurality of informed pulses of the electrical stimulation therapy (740). For example, IMD 110 may compare a value of a characteristic of the sensed ECAP to a target ECAP characteristic value and adjust the informed pulses and, in some examples, the control pulses to maintain the target ECAP characteristic vale. IMD 110 may deliver, via electrodes 130, the electrical stimulation therapy to spinal cord 120 of patient 105 according to the adjusted one or more parameter values (750).

As described herein, unlike that of the informing control pulses, IMD 110 may not sense the ECAP elicited by non-informing control pulses. For example, IMD 110 may control sensing circuitry 212 to be active following the delivery of the informing control pulses and IMD 110 may control sensing circuitry 212 to be inactive follow delivery of the non-informing control second pulses.

As described herein, in some examples, IMD 110 may adjust the pattern and/or ratio of informing to non-informing control pulses of the plurality of control pulses delivered by IMD 110 (720), e.g., while maintaining the frequency at which the plurality of control pulses are delivered. Additionally, or alternatively, IMD 110 may deliver the plurality of control pulses (720) such that informing control pulses have an interphase interval that is less than the interphase interval of the non-informing control pulses (e.g., as illustrated in FIG. 4). Additionally, or alternatively, IMD 110 may deliver the plurality of control pulses (720) such that non-informing control pulses alternate between a cathodic active first phase and an anodic active first phase according to a ratio.

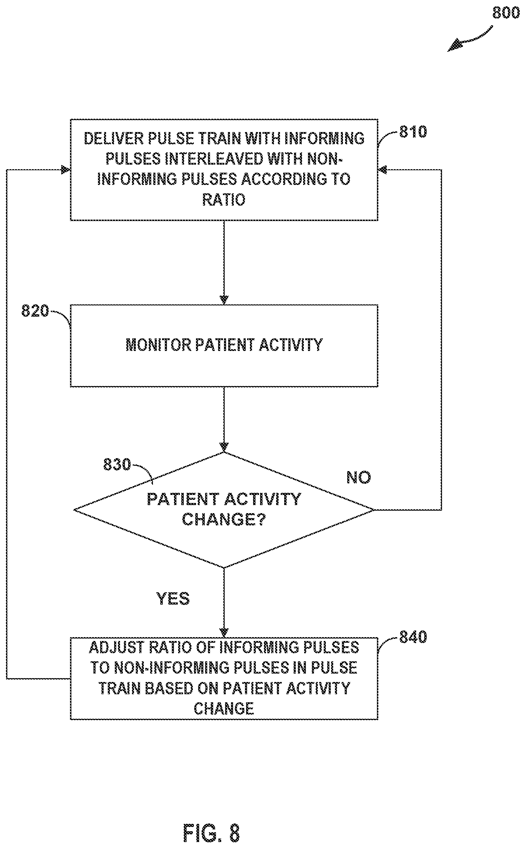

FIG. 8 is a flowchart illustrating an example operation 800 for delivery of control pulses in response to sensor input. For convenience, FIG. 8 is described with respect to IMD 200 of FIG. 2A. However, the techniques of FIG. 8 may be performed by different components of IMD 200 or by additional or alternative medical devices.

In the example of FIG. 8, processing circuitry 214 may deliver electrical stimulation including a train of control pulses including informing pulses and non-informing pulses (810). Processing circuitry 214 may deliver the train of control pulses according to a ratio of informing pulses to non-informing pulses. For example, the ratio may be 1:2 as shown for the train of control pulses 304 in FIG. 3. The electrical stimulation may also include informed pulses, such as, informed pulses 324 in FIG. 3, that are delivered in conjunction with the train of control pulses in the manner describe in FIG. 7.

Processing circuitry 214 monitors patient activity during the delivery of the electrical stimulation therapy (820). The patient activity may include changes in patient movement, switching to different postures, or any other type of activity which may cause the electrodes to move with respect to the target tissue (e.g., neurons).

If processing circuitry 214 does not detect any patient activity change ("NO" branch of block 830), processing circuitry 214 may then continue to deliver electrical stimulation therapy without adjusting the ratio of informing control pulse to non-informing control pulses (810). If processing circuitry 214 does detect a patient activity change ("YES" branch of block 830), processing circuitry 214 may update the ECAP test stimulation program so that the ratio is adjusted according to the patient activity change (840). For example, processing circuitry 214 may increase the ratio of informing pulses to non-informing pulses in the train of control pulses that are delivered in response to detecting an increase in patient activity. The frequency at which the control pulses are delivered may remain the same. In some examples, this increase in ratio may continue for a specified period of time or indefinitely. Conversely, the processing circuitry 214 may decrease the ratio of informing pulses to non-informing pulses in the train of control pulses that are delivered in response to detecting a decrease in patient activity. The frequency at which the control pulses are delivered may remain the same.

Processing circuitry 214 may then control the stimulation generator 211 to deliver the control pulses to the patient according to the adjusted ratio and continue delivering electrical stimulation therapy with the train of control pulses (810). In other examples, processing circuitry 214 may update an ECAP test stimulation program with an adjusted ratio in response to receiving a user input indicating that an activity has changed or will be changing soon. Alternatively, a patient may request an adjustment to the ratio if the patient believes that stimulation therapy is not adjusting to activity or posture changes in an adequate amount of time.

FIG. 9 is a flowchart illustrating an example operation 900 for delivery of control pulses in response to sensor input. For convenience, FIG. 9 is described with respect to IMD 200 of FIG. 2A. However, the techniques of FIG. 9 may be performed by different components of IMD 200 or by additional or alternative medical devices.

In the example of FIG. 9, processing circuitry 214 may deliver electrical stimulation including a train of control pulses including informing pulses and non-informing pulses, where the non-informing pulses alternate between a cathodic active first phase and an anodic active first phase, e.g., with a passive second phase (910). Processing circuitry 214 may deliver the train of control pulses according to a ratio of cathodic active first phase and an anodic active first phase for the non-informing pulses. For example, the ratio may be 1:1 as shown for the train of control pulses 504B, 504C, and 504E in FIG. 4. The electrical stimulation may also include informed pulses, such as, informed pulses 324 in FIG. 3, that are delivered in conjunction with the train of control pulses in the manner describe in FIG. 7.

Processing circuitry 214 monitors glutamate level of the patient during the delivery of the electrical stimulation therapy (920). The glutamate level may be monitored using any suitable glutamate sensor, such as an electrochemical or optical sensor.

If processing circuitry 214 does not detect any glutamate level change ("NO" branch of block 930), processing circuitry 214 may then continue to deliver electrical stimulation therapy without adjusting the ratio of cathodic active first phase and an anodic active first phase for the non-informing control pulses (910). If processing circuitry 214 does detect a glutamate level change ("YES" branch of block 930), processing circuitry 214 may update the ECAP test stimulation program so that the ratio of cathodic active first phase and an anodic active first phase for the non-informing pulses is adjusted according to the glutamate level change (940). For example, processing circuitry 214 may increase the ratio of cathodic active first phase and an anodic active first phase for the non-informing pulses in the train of control pulses that are delivered in response to detecting an increase in glutamate level. The frequency at which the control pulses are delivered may remain the same. In some examples, this increase in ratio may continue for a specified period of time or indefinitely. Conversely, the processing circuitry 214 may decrease the ratio of cathodic active first phase and an anodic active first phase for the non-informing pulses in the train of control pulses that are delivered in response to detecting a decrease in glutamate level. The frequency at which the control pulses are delivered may remain the same. Other candidate chemicals beyond glutamate that may be used as feedback in the described techniques may include Substance P, 5-HT, kinins, histamine, nerve growth factors, leukotrienes, nitric oxide and/or local pH changes. Some example substances may include those described by Yam, Mun Fei et al. "General Pathways of Pain Sensation and the Major Neurotransmitters Involved in Pain Regulation." International journal of molecular sciences vol. 19,8 2164. 24 Jul. 2018, doi:10.3390/ijms19082164. In general, one or more relevant biological processes may be measured by processing circuitry with suitable sensors and may adjust one or more parameters of non-informing and/or informing control pulses. It has been determined that stimulation with a symmetric, actively balanced signal may reduce glutamate secretion by the astrocytes when compared to passive recharged or predominant cathodic signals.

Processing circuitry 214 may then control the stimulation generator 211 to deliver the control pulses to the patient according to the adjusted ratio and continue delivering electrical stimulation therapy with the train of control pulses (910). In other examples, processing circuitry 214 may update an ECAP test stimulation program with an adjusted ratio in response to receiving a user input indicating that the ratio should be adjusted, e.g., either directly or indirectly.

Additionally, or alternatively, processing circuitry may adjust such a ratio based on an input other than that of a sensed glutamate level. For example, the polarity of the informing control pulses with a passive second phase may be governed by one or more characteristics of the ECAP elicited from the informing control pulses (e.g., any of the ECAP characteristics informing a closed-loop policy as described above such as slope, spectral content, amplitudes, latency, and/or the like), or by the detection of a certain concentration of a substance from a chemical sensor other than that of glutamate such as those substances described above.

As described above, in addition to or as an alternative to the delivery of informing and non-informing control pulses in the manner described above, in some examples, examples of the disclosure also relate to the delivery of pulse trains, such as a train of control pulses, following the sensing of electrical signals elicited by electrical stimulation. In the case of a train of informed pulses delivered following a control pulse, the informed pulse train may be configured to contribute to the therapy for a patient, e.g., by treating a patient condition, and may be adjusted based on the sensed electrical signal as described herein. As will be described below, the informed pulse train may include at least one first pulse at least partially interleaved with at least one second pulse, where the at least one first pulse includes a cathodic active first phase and a passive second phase, and where the at least one second pulse includes an anodic first phase and passive second phase. In some examples, the delivery of informed pulses in such a manner may be employed to limit stimulation artifacts and maintain electrochemical balance (e.g., at the tissue site at which the stimulation is delivered to the patient), without the battery capacity draw associated with a train of pulses having an active second (recharge) phase.

In some examples of the disclosure, the system may deliver electrical stimulation to a patient in which the informed pulses (such as informed pulses 324) are delivered as plurality of pulses after each informing control pulse. The plurality of informed pulses may be delivered with alternating polarity, where each pulse has an active first phase and a passive second phase.

FIG. 10 is an example timing diagram illustrating control pulses 550A and 550B, which may be the same or similar to that of, e.g., informing control pulses 304A and 304B. As show in FIG. 10, plurality of first informed pulses 552 and plurality of second informed pulses 554 are delivered between control pulses 550A and 550B and are at least partially interleaved with each other (e.g., shown as alternating every other pulse). First pulses 552 and second pulses 554 may be informed or otherwise controlled based on the signal evoked by informing control pulse 550A as described herein. First pulses 552 and second pulses 554 each have an active first phase and a passive second phase. However, the polarity of the active first phase of first pulses 552 is the opposite of the polarity of the active first phase of second pulses 554. For example, first pulses 552 may have an anodic first phase and second pulses 554 may have a cathodic first phase, or vice versa. A similar train of first and second pulses 552, 554 may be delivered following informed pulse 550B. The alternating polarity of first pulses 552 and second pulses 554 may reduce or eliminate residual charge in tissue that could affect the sensing of bioelectrical signals from the patient.

FIG. 11 is a timing diagram showing an example of first pulses 552 interleaved with second pulses 554 on a two by two basis (e.g., two first pulses 552 followed by two second pulses 554, and so forth. This two by two basis may be referred to as a partially interleaving basis or partially alternating polarity basis. FIG. 12 is a timing diagram showing an example of first pulses 552 interleaved with second pulses 554 on a one by one basis (e.g., one first pulse 552 followed by on second pulse 554, and so forth. First pulses 552 and second pulses 554 may be interleaved on any basis in this manner. In other examples, first pulses 552 and second pulses 554 may be delivered in equal numbers (e.g., balanced) or someone unequal numbers (e.g., unbalanced) and provide reduced residual charge compared with a pulse train having pulses of the same polarity.

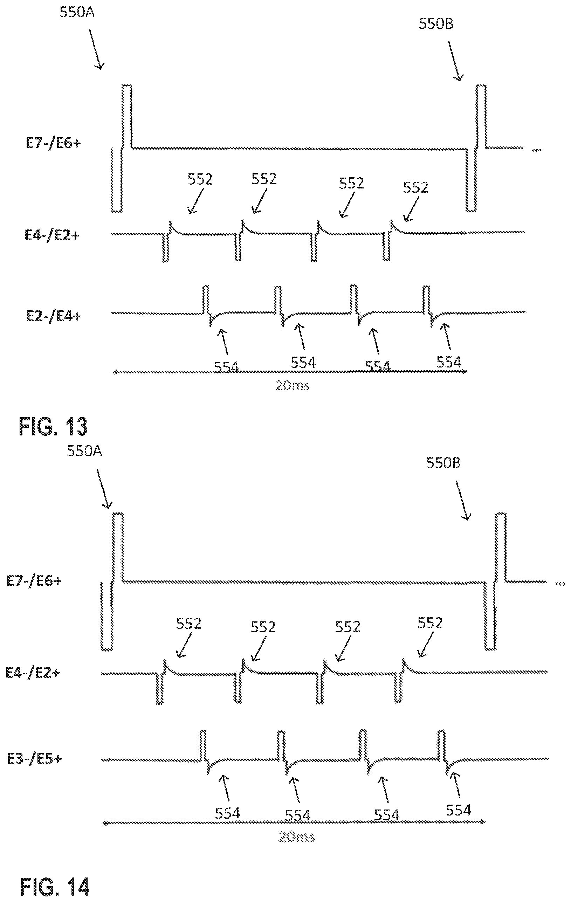

FIG. 13 is a timing diagram showing example electrode combinations and polarity used for the delivery of the electrical stimulation shown, e.g., in FIG. 10. As shown, informed pulse 550A, 550B may be delivered with an E7(-) and E6(+) electrode/polarity combination, first pulses 552 may be delivered with an E4(-) and E2(+) electrode/polarity combination, and second pulses 552 may be delivered with an E4(+) and E2(-) electrode/polarity combination. Accordingly, in some examples, first pulses 552 and second pulses 554 may be delivered with the same electrodes but with opposite polarities to deliver the described stimulation.

FIG. 14 is another timing diagram showing example electrode combinations and polarity used for the delivery of the electrical stimulation shown, e.g., in FIG. 10. As shown, informed pulse 550A, 550B may be delivered with an E7(-) and E6(+) electrode/polarity combination, first pulses 552 may be delivered with an E4(-) and E2(+) electrode/polarity combination, and second pulses 552 may be delivered with an E3(-) and E5(+) electrode/polarity combination. Accordingly, in some examples, first pulses 552 and second pulses 554 may be delivered with one or more different electrodes deliver the described stimulation.

In some examples of the disclosure, a stimulation pattern may be employed that allows for passive anodic stimulation while reducing the negative effects on biopotential sensing. This pattern may allow for more current draw savings than an active anodic system and more capability for biopotential sensing due to reduced charge build-up on electrodes. In other words, alternating polarity of delivered pulses may reduce or eliminate residual charge on electrodes that may affect sensing of biopotentials. Such a stimulation pattern may be delivered prior to sensing any suitable biopotential, e.g., such as an ECAP from an informed control pulse such as that described above.

By way of example, if 1000Hz stimulation is the desired frequency, the pattern would appear as shown in FIG. 11, where at every pulse, the electrode designated as the cathode and the electrode designated as the anode would flip. This may happen on every pulse, or not on every pulse, but after n number of pulses (example of n=2 shown FIG. 12).

This paradigm can also be employed in a time-interleaved fashion, where this stimulation pattern is delivered between pulses at a slower frequency, e.g., optimized for biopotential sensing. In an exemplary spinal cord stimulation where polymodal stimulation is intended, 50 Hz active anodic stimulation may be interleaved with 1000Hz passive anodic stimulation with alternating polarity. An example of this is shown graphically in FIG. 10, for example.

The other-time interleaved programs can be delivered on different electrodes than the alternating polarity stimulation. In the example above, for instance, the stimulation might be delivered to electrodes E7/E6 of an eight-electrode lead. Time-interleaved stimulation may be delivered between E4/E2. The amplitude, frequency, or pulse width of the alternating-polarity pulses may be modulated given the characteristics of the ECAP evoked from the stimulation complex with the active anodic phase. Such an examples is shown in FIG. 13. In yet other embodiments of the disclosure, the time-interleaved stimulation be delivered at a plurality of sites as long as it consists of alternating polarity with passive recharge (e.g., as shown in FIG. 14).

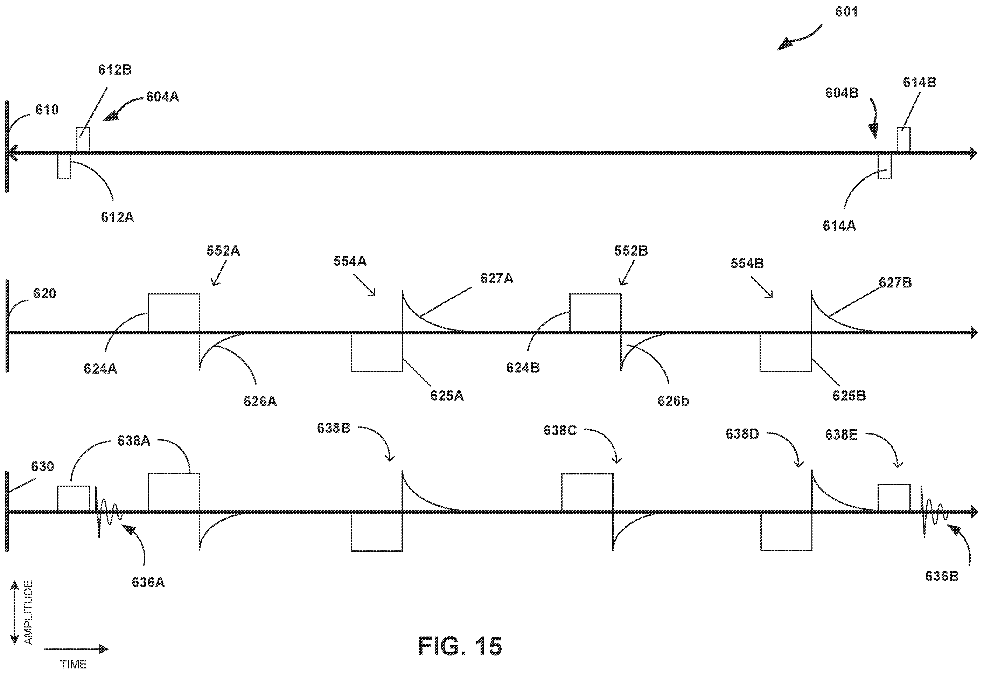

FIG. 15 is a timing diagram 601 illustrating one example of electrical stimulation pulses and respective sensed ECAPs according to some techniques of the disclosure. For convenience, FIG. 15 is described with reference to IMD 200 of FIG. 2A. The timing diagram 601 of FIG. 15 may be similar to that of the timing diagram 301 of FIG. 3 with some of the differences described below.

As illustrated, timing diagram 601 includes first channel 610, a plurality of control pulses 604A and 604B (collectively "control pulses 604"), second channel 620, a plurality of first informed pulses 552A and 552B (collectively "first pulses 552") including active first phases 624A and 624B (collectively "active first phases 624") and passive second recharge phases 626A and 626B (collectively "passive second recharge phases 626"), a plurality of second informed pulses 554A and 554B (collectively "second pulses 554") including active first phases 625A and 625B (collectively "active first phases 625") and passive second recharge phases 627A and 627B (collectively "passive second recharge phases 627"), third channel 630, a plurality of respective ECAPs 636A and 636B (collectively "ECAPs 636"), and a plurality of stimulation interference signals 638A-638E (collectively "stimulation interference signals 638").

First channel 610 is a time/voltage (and/or current) graph indicating the voltage (or current) of at least one electrode of electrodes 232, 234. In one example, the stimulation electrodes of first channel 610 may be located on the opposite side of the lead as the sensing electrodes of third channel 630. Control pulses 604 may be electrical pulses delivered to the spinal cord of the patient by at least one of electrodes 232, 234.

Unlike that of FIG. 3, control pulses 604 include only informing control pulses 604A and 604B, although other examples are contemplated in which control pulses 604 include both informing and non-informing control pulses like that described in FIG. 3. Informing control pulses 604A and 604B may be balanced biphasic square pulses with an interphase interval. In other words, each of informing control pulses 604A and 604B are shown with active first phase 612A and an active second phase 612B separated by an interphase interval (not labelled). For both of controls pulses 604A and 604B, the first phase 612A and second phase 612B have equal but opposite charges such that the remaining charge after the respective pulse is delivered is approximately zero. For example, informing control pulse 604A may have a negative voltage (e.g., cathodic voltage) for the same amount of time and amplitude that it has a positive voltage (e.g., anodic voltage). It is noted that the negative voltage phase may be before or after the positive voltage phase.

Control pulses 612 may be delivered according to ECAP test stimulation programs 218 stored in memory 250 of IMD 200, and ECAP test stimulation programs 218 may be updated according to user input via an external programmer and/or may be updated according to a signal from sensor 216. In one example, informing control pulses 604 may have a pulse width of less than approximately 300 microseconds (e.g., the total time of the positive phase, the negative phase, and the interphase interval is less than 300 microseconds). In another example, informing control pulses 604 may have a pulse width of approximately 150 µs for each phase of the bi-phasic pulse.

Second channel 620 is a time/voltage (and/or current) graph indicating the voltage (or current) of at least one electrode of electrodes 232, 234 for the informed pulses 552 and 554. In one example, the electrodes of second channel 620 may partially or fully share common electrodes with the electrodes of first channel 610 and third channel 630. First and second informed pulses 552, 554 may also be delivered by the same leads 230 that are configured to deliver control pulses 604. First and second informed pulses 552, 554 may be interleaved with control pulses 604, such that the two types of pulses are not delivered during overlapping periods of time. However, first and second informed pulses 552, 554 may or may not be delivered by exactly the same electrodes that deliver control pulses 604. First and second informed pulses 552, 554 may be monophasic pulses with pulse widths of greater than approximately 300 µs and less than approximately 1000 µs, e.g., where the pulse width is the duration of the first phase. In fact, first and second informed pulses 552, 554 may be configured to have longer pulse widths than control pulses 604. As illustrated in FIG. 15, first and second informed pulses 552, 554 may be delivered on channel 620. In some examples, first and second informed pulses 552, 554 may be a burst of interleaved stimulation with first informed pulses 552 being at least partially interleaved with second informed pulses 554. First and second informed pulses 552, 554 may be a burst of stimulation (or a train of pulses) delivered between control pulse 604A and control pulse 604B. The frequency at which the combination of first and second informed pulses 552, 554 is delivered may be greater than the frequency at which control pulses 604 are delivered.

Each pulse of first and second informed pulses 552, 554 includes a passive recharge phase. For example, each active phase 624, 625 of first and second informed pulses 552, 554 may be followed by a passive recharge phase 626, 627 to equalize charge on the stimulation electrodes. Unlike a pulse configured for active recharge, wherein remaining charge on the tissue following a stimulation pulse is instantly removed from the tissue by an opposite applied charge, passive recharge allows tissue to naturally discharge to some reference voltage (e.g., ground or a rail voltage) following the termination of the informed pulse. In some examples, the electrodes of the medical device may be grounded at the medical device body. In this case, following the termination of active phase 624, 625 of first and second informed pulses 552, 554, the charge on the tissue surrounding the electrodes may dissipate to the medical device, creating a rapid decay of the remaining charge at the tissue following the termination of the active phase 624, 625. This rapid decay is illustrated in passive recharge phases 626, 627. Passive recharge phases 626, 627 may have a duration in addition to the pulse width of the directly preceding active phase 624, 625. Informed pulses 552, 554 may be defined, and be a part of, one or more stimulation programs. Although each of informed pulses 552, 554 are illustrated as having the same parameter values (e.g., the same pulse width, amplitude, and pulse shape), some of informed pulses 552, 554 may have one or more parameters that have different values from each other.

In accordance with some examples of the disclosure, the train of informed pulses delivered by IMD 110 following ECAP 636A elicited by control pulse 604 in timing diagram 601 includes first informed pulses 552 at least partially interleaved with second information pulses 554, where first informed pulses 552 have active first phase 624 that is the opposite polarity of active first phase 625 of second informed pulses 554. As described above, each of first informed pulses 552 and second informed pulses 554 includes passive second phases 626 and 627, respectively. Such a stimulation pattern may limit stimulation artifacts and maintain electrochemical balance (e.g., at the tissue site at which the stimulation is delivered to the patient), without the battery capacity draw associated with a train of pulses having an active second (recharge) phase. The reduced residual charge that results from the delivery of informed pulses 552, 554 may be less deleterious to ECAP 636B elicited by control pulse 604B than that resulting from the delivery of similar informed pulses but all having active first phases of the same polarity.

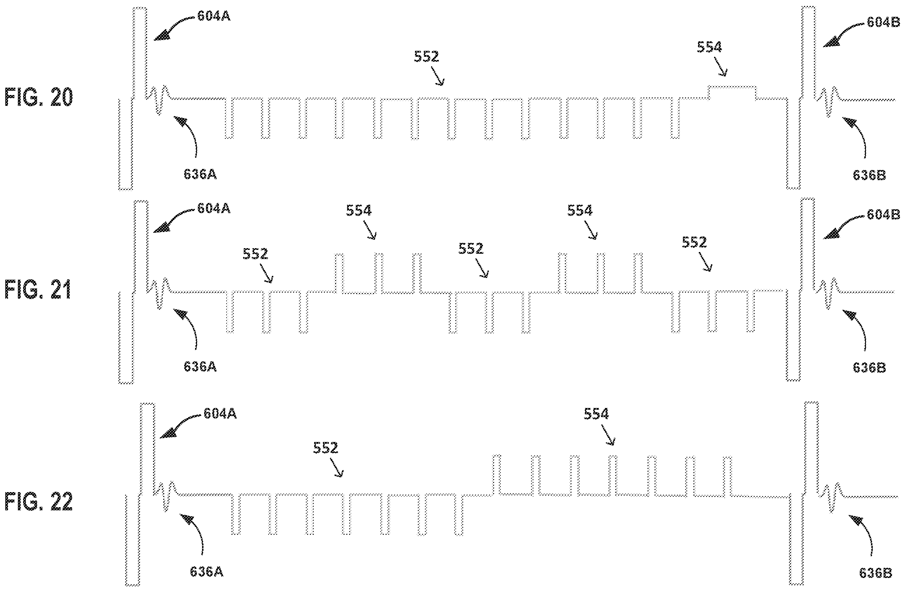

In the example of FIG. 15, first informed pulses 552 alternate with second informed pulses 554 on a one to one pulse basis. In other examples, other alternating patterns may be used, such as alternating on a two to two pulse basis, three by three basis, and/or where the first half of the train of informed pulses delivered between informing control pulses 604A and 604B may be anodic active first phase, and the second half of the train of informed pulses delivered between informing control pulses 604A and 604B may be cathodic active first phase, or vice versa. As one example, FIG. 22 is a timing diagram showing an example stimulation pattern in which, between control pulses 604A and 604B, the first half of the informed pulses 552 (seven total pulses) have a first polarity and the second half of the informed pulses 554 (seven total pulses) have the opposite polarity. While not shown, first and second pulses 552, 554 may have a second passive recharge phase following an active first phase. Furthermore, in some examples, one or more non-informing control pulses such as pulses 304BC, 304C in FIG. 3 may be delivered between informing control pulses 604A and 604B, e.g., such that first and second informed control pulses 552, 554 are at least partially interleaved with such non-informing control pulses.

While the example of FIG. 15 illustrates the number of first informed pulses 552 as being the same number of second informed pulses 554 between control pulses 604B and 604B (two of each type of pulse in FIG. 15), in other examples, the number of first pulses 552 may be different (e.g., less than or greater than) than the number of second pulses 554. As one example, FIG. 21 is a timing diagram showing an example stimulation pattern in which, between control pulses 604A and 604B, three sets of three first informed pulses 552 (nine total pulses) are alternated with two sets of three second informed pulses 554 (six total pulses) having the opposite polarity of first informed pulses 552, so that more first pulses 552 are delivered compared to second pulses 554 between control pulses 604A and 604B. While not shown, first and second pulses 552, 554 may have a second passive recharge phase following an active first phase.

As noted above, the amplitude and/or pulse with of first pulses 552 may be the same or different as that of second pulses 554. In some examples, IMD 110 may control the deliver more first pulses 552 than second pulses and/or with second pulses 554 having a greater pulse width and/or amplitude as the first pulses 552. In one example, IMD 100 may deliver a plurality of first pulses 552 and a single second pulse 554 (e.g., at the end of the train) between control pulses 604A and 604B, or vice versa. The single second pulse 554 may have the same or different amplitude and/or pulse width compared to that of the first pulses 552 in the pulse train. FIG. 20 is a timing diagram illustrating an example stimulation pattern in which a plurality of first pulses 552 (thirteen total) are first delivered followed by a single second pulse 554 between control pulses 604A and 604B, where the single second pulse 554 has a longer pulse width and lower amplitude compared to respective first pulses 552. While not shown, first and second pulses 552, 554 may have a second passive recharge phase following an active first phase.

Although not shown in FIG. 15, a burst of informed pulses like first and second informed pulses 552, 554 may be delivered following the sensing of ECAP 636B elicited by control pulse 604B. In some examples, the informed pulses following the sensing of ECAP 636B may be substantially the same as first and second informed pulses 552, 554 with alternating polarity active first phases and passive second phases. In other examples, the informed pulses following the sensing of ECAP 636B may be different than first and second informed pulses 552, 554, e.g., with the train of informed pulses having the same polarity active first phases and/or active second phases.

In some examples, first informed pulses 552 may be delivered using the same combination of electrodes as that used to deliver second informed pulses 554 (e.g., as shown in the diagram of FIG. 13 where a first electrode combination (E4- and E2+) is employed to deliver first pulses 552 and a second electrode combination (E4+ and E2-) is employed to deliver second pulses 554). In other examples, first informed pulses 552 may be delivered using an entirely different combination of electrodes as that used to deliver second informed pulses 554 (e.g., as shown in the diagram of FIG. 14 where a first electrode combination (E4- and E2+) is employed to deliver first pulses 552 and a second electrode combination (E3- and E5+) is employed to deliver second pulses 554). In other examples, first informed pulses 552 may be delivered using a combination of electrodes that has one or more electrodes in common as that used to deliver second informed pulses 554 but also one or more electrodes that are different from that used to deliver second informed pulses 554. Likewise, the electrodes combination used to deliver first pulses 552 and/or second pulses 554 may share one or more electrodes with the electrode combination used to deliver control pulses such as control pulses 604, or the electrode combination may not have any electrodes in common.

Third channel 630 is a time/voltage (and/or current) graph indicating the voltage (or current) of at least one electrode of electrodes 232, 234. In one example, the electrodes of third channel 630 may be located on the opposite side of the lead as the electrodes of first channel 610. ECAPs 636A and 636B may be sensed at electrodes 232, 234 from the spinal cord of the patient in response to informing control pulses 604A and 604B, respectively. ECAPs 636A and 636B are electrical signals which may propagate along a nerve away from the origination of informing control pulses 604A and 604B. In one example, ECAPs 636A and 636B are sensed by different electrodes than the electrodes used to deliver informing control pulses 604A and 604B. As illustrated in FIG. 15, ECAPs 336A and 336B may be recorded on third channel 330.

Stimulation interference signals 638A-638E (e.g., the artifact of the stimulation pulses) may be sensed by leads 230 and may be sensed during the same period of time as the delivery of control pulses 604 and informed pulses 552, 554. Since the interference signals may have a greater amplitude and intensity than ECAPs 336, any ECAPs arriving at IMD 200 during the occurrence of stimulation interference signals 638 may not be adequately sensed by sensing circuitry 212 of IMD 200. However, ECAPs 336 may be sufficiently sensed by sensing circuitry 212 because each ECAP 336 falls after the completion of each a control pulse 604 and before the delivery of the next informed pulse 552, 554. As illustrated in FIG. 3, stimulation interference signals 338 and ECAPs 336 may be recorded on channel 330.

In the example of FIG. 15, pulses 552, 554 may be controlled based the ECAP 636A elicited by informing control pulses 304A in the manner described herein. For example, stimulation pulses 552, 554 may be controlled based on ECAP 636A elicited by informing control pulse 604A and sensed by third channel 330, and another train of informed pulses may be delivered based on the ECAP 636B elicited by informing control pulse 604B and sensed by third channel 330. In such an example, stimulation pulses 552, 554 may be controlled to be substantially the same as each other (e.g., having the same pulse width, amplitude, and the like), and the train of informed pulses delivered after ECAP 636B may be controlled to be substantially the same as each other (e.g., having the same pulse width, amplitude, and the like).

In some examples, IMD 110 may sense an ECAP after the last pulse of a train of control pulses, e.g., where the train of control pulses includes at least some pulses having a passive recharge phase as show in FIG. 3, and control stimulation pulses 552, 554 based on the sensed ECAP elicited by the last control pulse. The last control pulse in the train of pulses may have an active recharge phase to reduce the overall time period of the first and second phases, e.g., as compared to control pulses with a passive recharge second phases. In other examples, IMD 110 may sense an ECAP after the last pulse of a train of pulses 552, 554, e.g., with the last pulse of the train eliciting the ECAP rather than delivering a control pulse to elicit an ECAP or other electrical signal. In such an example, IMD 110 control a subsequent train stimulation pulses 552, 554 based on the sensed ECAP elicited by the last pulse of the prior train of pulses 552, 554. The last control pulse in the train of pulses 552, 554 may have an active recharge phase to reduce the overall time period of the first and second phases, e.g., as compared to control pulses with a passive recharge second phases.

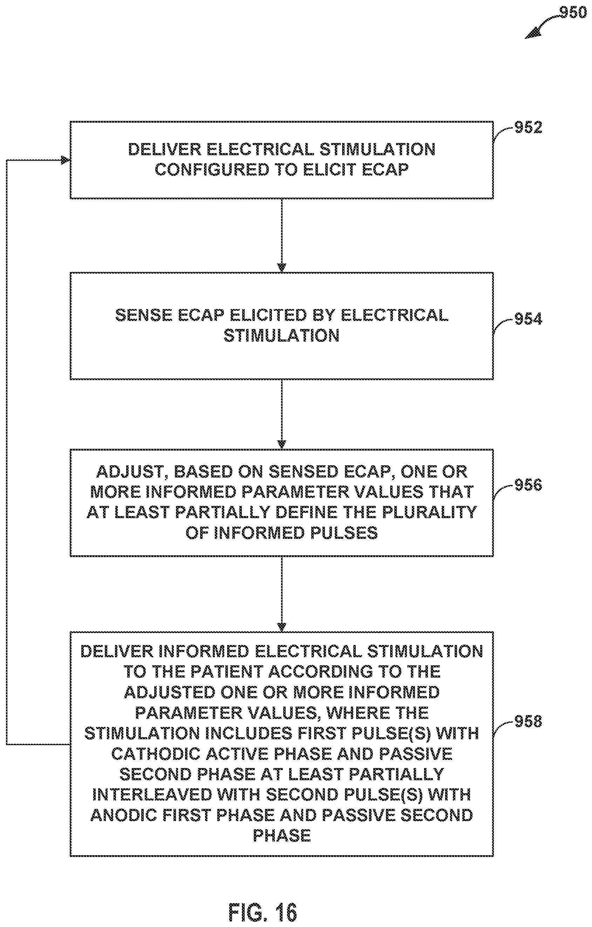

FIG. 16 is a flow diagram illustrating an example operation 950 for therapy delivery according to the techniques of this disclosure. For convenience, FIG. 16 is described with respect to IMD 110 of FIG. 1. However, the techniques of FIG. 16 may be performed by different components of IMD 110 or by additional or alternative medical devices. The example of FIG. 16 may be employed to deliver the example electrical stimulation represented by the timing diagram 601 of FIG. 15. For convenience, the example technique of FIG. 16 is described with regard to FIG. 15. However, the techniques of FIG. 16 may be employed to deliver any stimulation therapy that includes the delivery of a pulse train and the sensing of electrical signal(s) evoked by the delivery of stimulation therapy.

In the example of FIG. 16, IMD 110 may deliver electrical stimulation therapy to patient 105 that is configured to elicit an electrical signal (952) and then sense the elicited signal (954). Based on the sensed elicited signal, IMD 100 may then adjust one or more parameters values of informed pulses (956) and then deliver the informed pulses according to the adjust parameters, where the informed pulses include at least one first pulse having a cathodic active first phase and a passive second phase are at least partially interleaved with at least one second pulse having an anodic first phase and passive second phase (958). This process may be repeated on a closed loop basis as indicated in FIG. 16.

For example, IMD 110 may deliver informing control pulse 604A, which elicits ECAP 636A, and then sense the elicited ECAP 636A. IMD 110 may then adjust one or more parameters of informed pulses 552, 554 based on the sensed ECAP 636A, as described herein, and then deliver informed pulses 552, 554 according to the adjust parameters, where first informed pulses 552 having a cathodic active first phase and a passive second phase are at least partially interleaved with at least one second pulse 554 having an anodic first phase and passive second phase.

In some examples, IMD 110 may deliver first and second informed pulses 552, 554 in the manner described regardless of the ECAP 636A elicited by control pulse 604A and adjust other parameters of first and second informed pulses 552, 554 (e.g., pulse width, pulse amplitude, frequency, and/or the like) based on ECAP 636A. For example, IMD 110 may deliver first and second informed pulses 552, 554 in the manner described on a chronic basis between elicited ECAPs 636. In other examples, IMD 110 may deliver first and second informed pulses 552, 554 on a periodic basis over the course of treatment of a patient, e.g., with IMD 110 otherwise delivering informed pulses such as informed pulses 324 (FIG. 3). In some examples, IMD 110 may be configured to switch from informed pulses having all the same polarity active first phase and/or all having an active second phase to informed pulses such as first and second pulses 552, 554, where first informed pulses 552 having a cathodic active first phase and a passive second phase are at least partially interleaved with at least one second pulse 554 having an anodic first phase and passive second phase.

In some examples, the occurrence of one or more events may initiate the delivery of first and second pulses 552, 554, where first informed pulses 552 having a cathodic active first phase and a passive second phase are at least partially interleaved with at least one second pulse 554 having an anodic first phase and passive second phase. For example, IMD 110 may receive input from a user (e.g., clinician or patient) via programmer 300 indicating that informed pulses such as first and second pulses 552, 554 should be delivered (e.g., for a set period of time).

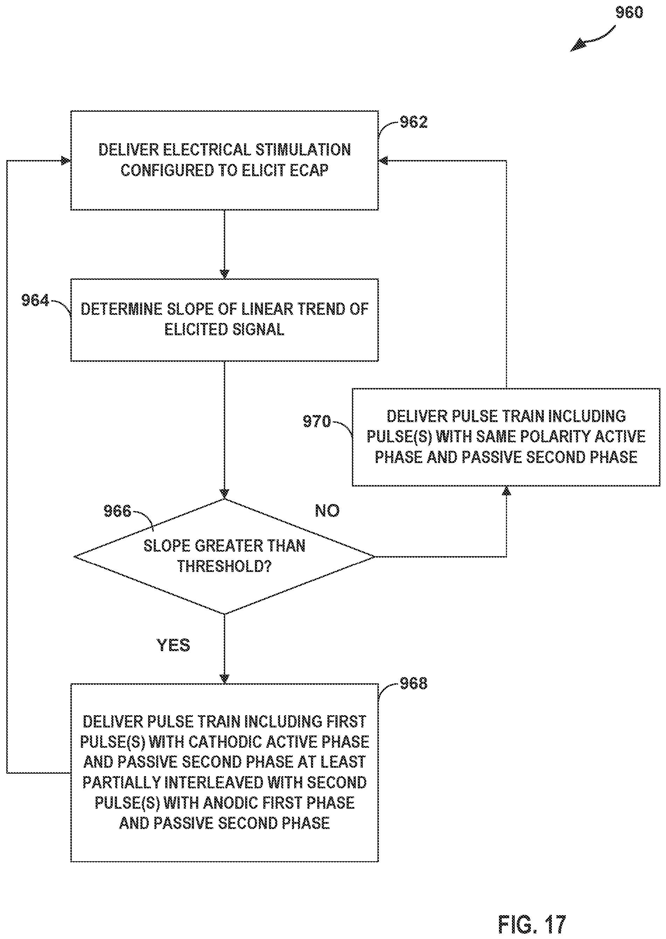

In other examples, IMD 110 may sense a parameter that indicates when the informed pulses should be delivered as first and second pulses, where first informed pulses 552 having a cathodic active first phase and a passive second phase are at least partially interleaved with at least one second pulse 554 having an anodic first phase and passive second phase. For example, IMD 110 may sense one or more parameters of ECAP 636A to initiate and/or suspend the delivery of first and second informed pulses 552, 554 following the sensing of ECAP 636A. Example parameters of ECAPs 636 that may be used as feedback to adjust the delivery of informed pulses so that the informed pulses include may include first informed pulses 552 having a cathodic active first phase and a passive second phase are at least partially interleaved with at least one second pulse 554 having an anodic first phase and passive second phase include a linear or non-linear slope of an elicited ECAP or the associated stimulation artifact (e.g., as shown in FIG. 17). As described above, for a sensed signal elicited by a control pulse, if the calculated artifact slope is an excess of a pre-determined threshold value, an algorithmic choice may be made by IMD 110 to take further steps to reduce the trend as described herein, e.g., by delivering a train of pulses with alternating polarity of an active first phase and a second passive phase.

FIG. 17 is a flow diagram illustrating an example operation 960 for therapy delivery according to the techniques of this disclosure. For convenience, FIG. 17 is described with respect to IMD 110 of FIG. 1. However, the techniques of FIG. 17 may be performed by different components of IMD 110 or by additional or alternative medical devices. The example of FIG. 17 may be employed to deliver the example electrical stimulation represented by the timing diagram 601 of FIG. 15. For convenience, the example technique of FIG. 17 is described with regard to FIG. 15. However, the techniques of FIG. 16 may be employed to deliver any stimulation therapy that includes the delivery of a pulse train and the sensing of electrical signal(s) evoked by the delivery of stimulation therapy.

As shown in FIG. 17, IMD 110 may deliver control pulse 604A to elicit ECAP 636A (962). IMD 10 may sense ECAP 636A and, based on the sensed signal, determine the slope of the linear trend for ECAP 636A (964). The techniques described above may be used by IMD 110, or other suitable technique, may be used to determine the slope of the linear (or non-linear in other examples) trend of ECAP 636A. The slope of the linear trend of ECAP 636A may be indicative of an undesirable effect from the delivery of prior informed pulses without alternating polarity first phases on the sensing of ECAP 636A. The greater the slope of linear trend may indicate a greater residual charge on electrodes and an undesired effect on the sensing of ECAP 636A by the prior informed pulses. Accordingly, if the slope of linear trend is relatively high, it may be desirable to modify the informed pulses delivered after sensing ECAP 636A to include a pulse train including first informed pulses 552 having a cathodic active first phase and a passive second phase at least partially interleaved with at least one second pulse 554 having an anodic first phase and passive second phase. As described herein, the delivery of informed pulses in such a manner may limit improve and/or maintain electrochemical balance (e.g., at the tissue site at which the stimulation is delivered to the patient). Additionally, or alternatively, without the battery capacity draw associated with a train of pulses having an active second (recharge) phase, the overall energy used to delivery such informed pulses may also be reduced.

Accordingly, as shown in FIG. 17, IMD 110 may then compare the determined slope of linear trend of ECAP 636A to a threshold value (966). The threshold value may be preprogrammed, e.g., by a clinician or other user, and may reflect a maximum value for the slope of linear trend of an ECAP before the slope of liner trends indicates that the sensing of ECAP 636A has been undesirably influenced by the delivery of informed pulses delivered prior to control pulse 604A. If IMD 110 determines that the determined slope of linear trend is greater than the threshold ("YES" in FIG. 17), then IMD 110 may deliver a train of informed pulses including first informed pulses 552 having a cathodic active first phase and a passive second phase at least partially interleaved with at least one second pulse 554 having an anodic first phase and passive second phase (968). Conversely, if IMD 110 determines that the determined slope of linear trend is less than or equal to the threshold ("NO" in FIG. 17), then IMD 110 may deliver a train of informed pulses that do not alternate in polarity and that do not have passive second phases (e.g., a train of pulses all having the same polarity active first phase and/or all having an active second recharge phase) (970). As shown in FIG. 17, this process may be carried out in a closed loop manner.

While the example of FIG. 17 is described in terms of adjusting informed pulses 552, 554 to have alternating polarity active first phases based on the determined slope of linear trend being greater than a threshold value, other similar examples are contemplated. For example, the parameter does not need to be a linear trend but may be a non-linear trend displaying some variation in the previously sensed ECAPs. In some examples, IMD 110 may initiate the delivery of alternating polarity active first phases pulses based on a linear or non-linear variation of one or more aspects of previously sensed ECAPs being greater than some threshold amount. Further, the determined trend may be for a single elicited electrical signal or may be the average trend for multiple electrical signals, e.g., elicited on a sequential basis.

Additionally, or alternatively, IMD 110 may determine the slope of a linear or non-linear trend for each of a plurality of sensed ECAPs. If IMD 110 determines that the slope is increasing over time for the sensed ECAPs, IMD 110 may deliver a train of informed pulses including first informed pulses 552 having a cathodic active first phase and a passive second phase at least partially interleaved with at least one second pulse 554 having an anodic first phase and passive second phase (968). Conversely, if IMD 110 determines that the slope is decreasing or remaining substantially constant for the sensed ECAPs (or is increasing below a threshold rate), then IMD 110 may deliver a train of informed pulses that do not alternate in polarity and that do not have passive second phases (e.g., a train of pulses all having the same polarity active first phase and/or all having an active second recharge phase) (970). As shown in FIG. 17, this process may be carried out in a closed loop manner. A similar closed loop feedback technique may be used to initiate the delivery of a train of informed pulses including first informed pulses 552 having a cathodic active first phase and a passive second phase at least partially interleaved with at least one second pulse 554 having an anodic first phase and passive second phase when IMD 110 determines the level of power source 355 is below a threshold amount, e.g., to reduced power consumption by using a second passive recharge phase for informed pulses 552, 554.

FIGS. 18 and 19 are example timing diagrams for two different electrical stimulation patterns. In each example, the electrical signal 636 elicited by control pulse 604B is measured approximately 45 different times (45 samples) on a serial basis at a frequency of 25 kHz. Accordingly, every 40 microseconds, a new measurement (sample) is acquired, the combination of which is shown in the plot on the right side of FIGS. 18 and 19 and reveals the complete ECAP and associated artifact over about 2 milliseconds (e.g., 50 samples times 40 milliseconds). In FIG. 18, following control pulse 604A, a train of informed pulses 992 having the same polarity active first phases and passive second recharge phases (not shown) are delivered by an IMD such as IMD 110. As described above, in FIG. 18 an ECAP is present from samples 5 to 15. The slope of the remaining portion of the recording-from samples 15 to 45-may be determined by fitting to an equation of form (ECAP) = (Artifact Slope)(Sample) + (ECAP Intercept). In FIG. 19, following control pulse 604A, a train of informed pulses 994 having the alternating polarity active first phases and passive second recharge phases (not shown) are delivered by an IMD such as IMD 110. As shown, following the delivery of control pulse 604B, the slope of linear trend 996 (about 60 uV) in FIG. 18 is greater than the slope of linear trend 998 in FIG. 19. This demonstrates an example in which the delivery of a pulse train including first informed pulses 552 having a cathodic active first phase and a passive second phase at least partially interleaved with at least one second pulse 554 having an anodic first phase and passive second phase may reduce the slope of linear trend for a ECAP 636 elicited by control pulse 604B after delivery of the train of informed pulses 994 compared to that of train of informed pulses 992 delivered with all the same polarity active first phases. This may be a result of a reduced amount of remaining charge in the tissue from the delivery of pulses 994 compared to pulses 992.

The examples of FIGS. 18 and 19 are shown as including control pulses 604 that elicit ECAPs such as ECAP 636 separate from informed pulses 992 and 994. In other examples, the ECAPs may be elicited by pulses 992 and 994 without the delivery of separate control pulses, e.g., with the last pulse in the train of pulses eliciting an ECAP. In such an example, the last pulse in the train of pulses 992 and 994 may have an active recharge second phase to reduce the time period when the sensing of the ECAP may be initiated without being interfered with by the second phase of the last pulse. Multiple different type of pulses may be delivered between sensing of elicited electrical signals, e.g., with different electrode combination and/or different amplitudes. The different types of pulses may be delivered by employing different programs to define the respective types of pulses.

Although the examples of FIGS. 10-19 are described with respect to control pulses and informed pulses, alternating polarity of stimulation pulses may be used in other scenarios. For example, the system may deliver a train of pulses followed by a sensing period. The train of pulses may have the same amplitude, pulse width, and frequency, or differ in one or more of these parameters. In any case, the train of pulses may alternate polarity of the pulses in the train in some manner, such as either every other pulse, a balanced number of pulses for each polarity, or an unbalanced (planned or random) number of pulses for each polarity. After the train of pulses has been delivered, the system can sense a physiological signal. The alternating polarity of pulses may reduce the amount of remaining charge in tissue immediately prior to sensing the physiological signal.

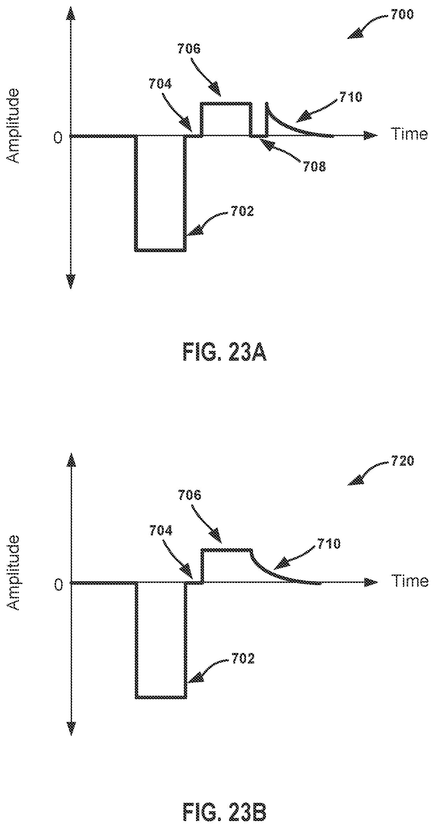

FIGS. 23A and 23B are timing diagrams illustrating phases of example electrical stimulation pulses 700 and 720 accordance with some examples of this disclosure. As described herein, stimulation pulses generally include an active first phase and a second phase, where the second phase can be referred to as a "recharge" phase. This recharge phase may be an active phase or a passive phase. However, in some examples, the recharge phase may by complex and include both an active portion and a passive portion (e.g., one or more of each of an active phase or a passive phase). These active and passive portions of the recharge phase may be separated by a delay or immediately follow one another without any delay. In addition, either the active portion or passive portion may be delivered first in the sequence.

As shown in FIG. 23A, stimulation pulse 700 includes first active phase 702. The first active phase 702 may act as the active stimulation phase that is perceived by tissue. The recharge phase is made up of active recharge portion 706 and passive recharge portion 710 which are separated by delay 708. In this manner, the system will deliver the recharge phase with the active recharge portion 706 occurring first and deliver the passive recharge portion 710 after the delay 708. A benefit to using both the active recharge portion 706 and passive recharge portion 710 is that the overall recharge phase consumes less power than a fully active recharge phase while also removing charge from the electrodes faster than a fully passive recharge phase. Delay 708 can be set to any duration appropriate for the delivered stimulation and/or the timing of any following stimulation pulses.

Stimulation pulse 720 of FIG. 23A is similar to stimulation pulse 700 of FIG. 23A. The first active phase 702 may still act as the active stimulation phase that is perceived by tissue. However, the recharge phase is made up of active recharge portion 706 which is immediately followed by passive recharge portion 710 without any separation or delay. This process may reduce the overall time needed to complete the recharge phase of stimulation pulse 720. In other examples, the system may deliver the passive recharge portion of any recharge phase first and then follow by delivering the active recharge portion.

The techniques described in this disclosure may be implemented, at least in part, in hardware, software, firmware or any combination thereof. For example, various aspects of the described techniques may be implemented within one or more processors or processing circuitry, including one or more microprocessors, digital signal processors (DSPs), application specific integrated circuits (ASICs), field programmable gate arrays (FPGAs), or any other equivalent integrated or discrete logic circuitry, as well as any combinations of such components. The term "processor" or "processing circuitry" may generally refer to any of the foregoing logic circuitry, alone or in combination with other logic circuitry, or any other equivalent circuitry. A control unit comprising hardware may also perform one or more of the techniques of this disclosure.

Such hardware, software, and firmware may be implemented within the same device or within separate devices to support the various operations and functions described in this disclosure. In addition, any of the described units, circuits or components may be implemented together or separately as discrete but interoperable logic devices. Depiction of different features as circuits or units is intended to highlight different functional aspects and does not necessarily imply that such circuits or units must be realized by separate hardware or software components. Rather, functionality associated with one or more circuits or units may be performed by separate hardware or software components or integrated within common or separate hardware or software components.

The techniques described in this disclosure may also be embodied or encoded in a computer-readable medium, such as a computer-readable storage medium, containing instructions that may be described as non-transitory media. Instructions embedded or encoded in a computer-readable storage medium may cause a programmable processor, or other processor, to perform the method, e.g., when the instructions are executed. Computer readable storage media may include random access memory (RAM), read only memory (ROM), programmable read only memory (PROM), erasable programmable read only memory (EPROM), electronically erasable programmable read only memory (EEPROM), flash memory, a hard disk, a CD-ROM, a floppy disk, a cassette, magnetic media, optical media, or other computer readable media.

Various examples have been described.

The scope of the present invention is defined by the following claims.

## Claims

1. A system comprising:
sensing circuitry (212);
a stimulation generator (211); and
processing circuitry (214), the processing circuitry (214) configured to:
control the stimulation generator (211) to deliver a pulse train of control pulses to a patient;
control the stimulation generator (211) to deliver a pulse train of informed pulses to a patient different from the control pulses in one or more aspects, wherein the pulse train of informed pulses is at least partially interleaved with the pulse train of control pulses, wherein the pulse train of informed pulses comprising a plurality of first pulses and a plurality of second pulses, wherein respective first pulses of the plurality of first pulses are interleaved with respective second pulses of the plurality of second pulses on a one to one pulse basis, wherein the first pulses include a cathodic active first phase and a passive second phase, and wherein the second pulses include an anodic first phase and passive second phase, and
sense, via the sensing circuitry (212), an electrical signal elicited by electrical stimulation delivered to the patient from the stimulation generator.

2. The system of claim 1, wherein the plurality of first pulses have the same amplitude as the plurality of second pulses.

3. The system of any one of claims 1 or 2, wherein the processing circuitry (214) is configured to determine a trend of a sensed electrical signal elicited by delivery of electrical stimulation, and control the stimulation generator to deliver the pulse train of informed pulses to the patient based on the sensed trend.

4. The system of claim 3, wherein the processing circuitry (214) is configured to:
determine a slope of the trend of the sensed electrical signal elicited by the delivery of the electrical stimulation;
determine the slope of the trend is greater than a threshold value; and
control the stimulation generator to deliver the pulse train of informed pulses to the patient based on the determination that the slope of the trend is greater than the threshold value.

5. The system of any one of claim 1-4, wherein the at least one first pulse is delivered by a first combination of electrodes and the at least one second pulse is delivered by a second combination of electrodes.

6. The system of claim 5, wherein the first combination of electrodes and the second combination of electrodes have at least one common electrode.

7. The system of claim 1, wherein the first combination of electrodes is different than the second combination of electrodes.

## Patentansprüche

1. System, umfassend:
Erfassungsschaltung (212);
einen Stimulationserzeuger (211); und
Verarbeitungsschaltung (214), wobei die Verarbeitungsschaltung (214) konfiguriert ist zum:
Steuern des Stimulationserzeugers (211), um eine Impulsfolge von Steuerimpulsen an einen Patienten abzugeben;
Steuern des Stimulationserzeugers (211), um eine Impulsfolge von informierten Impulsen an einen Patienten abzugeben, die sich in einem oder mehreren Aspekten von den Steuerimpulsen unterscheiden, wobei die Impulsfolge von informierten Impulsen mindestens teilweise mit der Impulsfolge von Steuerimpulsen verschachtelt ist, die Impulsfolge von informierten Impulsen umfassend eine Vielzahl von ersten Impulsen und eine Vielzahl von zweiten Impulsen, wobei jeweilige erste Impulse der Vielzahl von ersten Impulsen mit jeweiligen zweiten Impulsen der Vielzahl von zweiten Impulsen auf einer Eins-zu-Eins-Impulsbasis verschachtelt sind, wobei die ersten Impulse eine kathodische aktive erste Phase und eine passive zweite Phase einschließen, und wobei die zweiten Impulse eine anodische erste Phase und eine passive zweite Phase einschließen, und
Erfassen, über die Erfassungsschaltung (212), eines elektrischen Signals, das durch elektrische Stimulation hervorgerufen wird, die von dem Stimulationserzeuger an den Patienten abgegeben wird.

2. System nach Anspruch 1, wobei die Vielzahl von ersten Impulsen die gleiche Amplitude wie die Vielzahl von zweiten Impulsen aufweist.

3. System nach einem der Ansprüche 1 oder 2, wobei die Verarbeitungsschaltung (214) konfiguriert ist, um einen Trend eines erfassten elektrischen Signals zu bestimmen, das durch Abgabe elektrischer Stimulation hervorgerufen wird, und den Stimulationserzeuger zu steuern, um die Impulsfolge von informierten Impulsen basierend auf dem erfassten Trend an den Patienten abzugeben.

4. System nach Anspruch 3, wobei die Verarbeitungsschaltung (214) konfiguriert ist zum:
Bestimmen einer Steigung des Trends des erfassten elektrischen Signals, das durch die Abgabe der elektrischen Stimulation hervorgerufen wird;
Bestimmen, dass die Steigung des Trends größer als ein Schwellenwert ist; und
Steuern des Stimulationserzeugers, um die Impulsfolge von informierten Impulsen an den Patienten abzugeben, basierend auf der Bestimmung, dass die Steigung des Trends größer als der Schwellenwert ist.

5. System nach einem der Ansprüche 1 bis 4, wobei der mindestens eine erste Impuls durch eine erste Kombination von Elektroden abgegeben wird und der mindestens eine zweite Impuls durch eine zweite Kombination von Elektroden abgegeben wird.

6. System nach Anspruch 5, wobei die erste Kombination von Elektroden und die zweite Kombination von Elektroden mindestens eine gemeinsame Elektrode aufweisen.

7. System nach Anspruch 1, wobei sich die erste Kombination von Elektroden von der zweiten Kombination von Elektroden unterscheidet.

## Revendications

1. Système comprenant :
un ensemble de circuits de détection (212) ;
un générateur de stimulation (211) ; et
un ensemble de circuits de traitement (214), l'ensemble de circuits de traitement (214) est configuré pour :
commander le générateur de stimulation (211) pour délivrer un train d'impulsions de commande à un patient ;
commander le générateur de stimulation (211) pour délivrer un train d'impulsions informées à un patient différentes des impulsions de commande selon un ou plusieurs aspects, dans lequel le train d'impulsions informées est au moins partiellement entrelacé avec le train d'impulsions de commande, dans lequel le train d'impulsions informées comprenant une pluralité de premières impulsions et une pluralité de secondes impulsions, dans lequel des premières impulsions respectives de la pluralité de premières impulsions sont entrelacées avec des secondes impulsions respectives de la pluralité de secondes impulsions sur la base d'une impulsion pour une, dans lequel les premières impulsions comprennent une première phase active cathodique et une seconde phase passive, et dans lequel les secondes impulsions comprennent une première phase anodique et une seconde phase passive, et
détecter, par l'intermédiaire de l'ensemble de circuits de détection (212), un signal électrique induit par une stimulation électrique délivrée au patient par le générateur de stimulation.

2. Système selon la revendication 1, dans lequel la pluralité de premières impulsions a la même amplitude que la pluralité de secondes impulsions.

3. Système selon l'une quelconque des revendications 1 ou 2, dans lequel l'ensemble de circuits de traitement (214) est configuré pour déterminer une tendance d'un signal électrique détecté provoqué par la délivrance d'une stimulation électrique, et commander le générateur de stimulation pour délivrer le train d'impulsions informées au patient sur la base de la tendance détectée.

4. Système selon les revendications 3, dans lequel l'ensemble de circuits de traitement (214) est configuré pour :
déterminer une pente de la tendance du signal électrique détecté induit par la délivrance de la stimulation électrique ;
déterminer que la pente de la tendance est supérieure à une valeur de seuil ; et
commander le générateur de stimulation pour délivrer le train d'impulsions d'impulsions informées au patient sur la base de la détermination selon laquelle la pente de la tendance est supérieure à la valeur de seuil.

5. Système selon l'une quelconque des revendications 1 à 4, dans lequel l'au moins une première impulsion est délivrée par une première combinaison d'électrodes et l'au moins une seconde impulsion est délivrée par une seconde combinaison d'électrodes.

6. Système selon la revendication 5, dans lequel la première combinaison d'électrodes et la seconde combinaison d'électrodes ont au moins une électrode commune.

7. Système selon la revendication 1, dans lequel la première combinaison d'électrodes est différente de la seconde combinaison d'électrodes.
